# EUROPÄISCHE PATENTSCHRIFT

(11) **EP 1 800 697 B1**
(45) Veröffentlichungstag und Bekanntmachung des Hinweises auf die Patenterteilung: **14.04.2010**
(21) Anmeldenummer: 07000343.9
(22) Anmeldetag: 05.06.2002
(51) Int. Cl.: C07K 14/11, C07K 14/47, A61K 48/00, A61K 39/00, A61K 39/145, A61P 11/00, A61P 43/00

(54) **Stabilisierte mRNA mit erhöhtem G/C-Gehalt für die Gentherapie**
Stabilised mRNA with increased G/C-content for use in gene therapy
ARNm avec un contenu G/C augmenté pour l'utilisation dans la thérapie génique

(30) Priorität: 05.06.2001 DE 10127283
(43) Veröffentlichungstag der Anmeldung: 27.06.2007
(62) Teilanmeldung aus: 05004938.6
(73) Patentinhaber: CureVac GmbH, 72076 Tübingen (DE)
(72) Erfinder: von der Mülbe, Florian, 72070 Tübingen (DE); Pascolo, Steve, 72072 Tübingen (DE); Hoerr, Ingmar, 72070 Tübingen (DE)
(74) Vertreter: Graf von Stosch, Andreas

(56) Entgegenhaltungen:
- EP-A- 1 083 232
- WO-A-01/04313
- WO-A-02/08435
- WO-A-97/48370
- WO-A-98/34640
- WO-A-99/14346
- WO-A-99/20774
- WO-A-03/051401
- US-B1- 6 214 804
- LATHE R: "SYNTHETIC OLIGONUCLEOTIDE PROBES DEDUCED FROM AMINO ACID SEQUENCE DATA. THEORETICAL AND PRACTICAL CONSIDERATIONS" JOURNAL OF MOLECULAR BIOLOGY, LONDON, GB, Bd. 183, Nr. 1, 5. Mai 1985 (1985-05-05), Seiten 1-12, XP000674208 ISSN: 0022-2836
- HOERR INGMAR ET AL: "In vivo application of RNA leads to induction of specific cytotoxic T lymphocytes and antibodies" EUROPEAN JOURNAL OF IMMUNOLOGY, Bd. 30, Nr. 1, Januar 2000 (2000-01), Seiten 1-7, XP002243972 ISSN: 0014-2980
- HOLCIK M ET AL: "Four highly stable eukaryotic mRNAs assemble 3' untranslated region RNA-protein complexes sharing cis and trans components" PROCEEDINGS OF THE NATIONAL ACADEMY OF SCIENCES OF USA, NATIONAL ACADEMY OF SCIENCE, WASHINGTON, DC, US, Bd. 94, Nr. 6, 1997, Seiten 2410-2414, XP002243974 ISSN: 0027-8424

### SEQUENCE LISTING

<110> CureVac GmbH
<120> Pharmazeutische Zusammensetzung, enthaltend eine stabilisierte und für die Translation in ihren codierenden Bereichen optimierte mRNA
<130> CU01P003WOEPT2
<150> PCT/EP02/06180
   <151> 2002-06-05
<160> 13
<170> PatentIn Ver. 2.1
<210> 1
   <211> 774
   <212> DNA
   <213> Influenza virus
<220>
   <223> Influenza-Matrix: Wildtyp-Gen (zum Vergleich)
<220>
   <223> Startcodon: atg (Nucleotide 11 bis 13), Stopcodon: tga (Nucleotide 767 bis 769 )
<400> 1
   agatctaaag atgagtcttc taaccgaggt cgaaacgtac gttctctcta tcatcccgtc 60
   aggccccctc aaagccgaga tcgcacagag acttgaagat gtctttgcag ggaagaacac 120
   cgatcttgag gttctcatgg aatggctaaa gacaagacca atcctgtcac ctctgactaa 180
   ggggatttta ggatttgtgt tcacgctcac cgtgcccagt gagcgaggac tgcagcgtag 240
   acgctttgtc caaaatgccc ttaatgggaa cggggatcca aataacatgg acaaagcagt 300
   taaactgtat aggaagctca agagggagat aacattccat ggggccaaag aaatctcact 360
   cagttattct gctggtgcac ttgccagttg tatgggcctc atatacaaca ggatgggggc 420
   tgtgaccact gaagtggcat ttggcctggt atgtgcaacc tgtgaacaga ttgctgactc 480
   ccagcatcgg tctcataggc aaatggtgac aacaaccaac ccactaatca gacatgagaa 540
   cagaatggtt ttagccagca ctacagctaa ggctatggag caaatggctg gatcgagtga 600
   gcaagcagca gaggccatgg aggttgctag tcaggctagg caaatggtgc aagcgatgag 660
   aaccattggg actcatccta gctccagtgc tggtctgaaa aatgatcttc ttgaaaattt 720
   gcaggcctat cagaaacgaa tgggggtgca gatgcaacgg ttcaagtgaa ctag       774
<210> 2
   <211> 252
   <212> PRT
   <213> Influenza virus
<400> 2
   Met Ser Leu Leu Thr Glu Val Glu Thr Tyr Val Leu Ser Ile Ile Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Val Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Asn Gly Asn Gly Asp Pro Asn Asn Met Asp Lys Ala
                   85                  90                  95
   Val Lys Leu Tyr Arg Lys Leu Lys Arg Glu Ile Thr Phe His Gly Ala
               100                 105                 110
   Lys Glu Ile Ser Leu Ser Tyr Ser Ala Gly Ala Leu Ala Ser Cys Met
           115                 120                 125
   Gly Leu Ile Tyr Asn Arg Met Gly Ala Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Gln
           195                 200                 205
   Ala Arg Gln Met Val Gln Ala Met Arg Thr Ile Gly Thr His Pro Ser
       210                 215                 220
   Ser Ser Ala Gly Leu Lys Asn Asp Leu Leu Glu Asn Leu Gln Ala Tyr
   225                 230                 235                 240
   Gln Lys Arg Met Gly Val Gln Met Gln Arg Phe Lys
                   245                 250
<210> 3
   <211> 775
   <212> DNA
   <213> Artificial Sequence
<220>
   <223> Description of Artificial Sequence: Influenza-Matrix: Gen mit erhöhtem G/C-Gehalt
<220>
   <223> Startcodon: atg (Nucleotide 11 bis 13), Stopcodon: tga (Nucleotide 767 bis 769)
<400> 3
   agatctaaag atgagcctgc tgaccgaggt ggagacctac gtgctgagca tcatccccag 60
   cggccccctg aaggccgaga tcgcccagag gctggaggac gtgttcgccg gcaagaacac 120
   cgacctggag gtgctgatgg agtggctgaa gaccaggccc atcctgagcc ccctgaccaa 180
   gggcatcctg ggcttcgtgt tcaccctgac cgtgcccagc gagcgcggcc tgcagcgccg 240
   ccgcttcgtg cagaacgccc tgaacggcaa cggcgacccc aacaacatgg acaaggccgt 300
   gaagctgtac aggaagctga agagggagat caccttccac ggcgccaagg agatcagcct 360
   gagctacagc gccggcgccc tggccagctg catgggcctg atctacaaca ggatgggcgc 420
   cgtgaccacc gaggtggcct tcggcctggt gtgcgccacc tgcgagcaga tcgccgacag 480
   ccagcaccgc agccacaggc agatggtgac caccaccaac cccctgatca ggcacgagaa 540
   caggatggtg ctggccagca ccaccgccaa ggccatggag cagatggccg gcagcagcga 600
   gcaggccgcc gaggccatgg aggtggccag ccaggccagg cagatggtgc aggccatgag 660
   gaccatcggc acccacccca gcagcagcgc cggcctgaag aacgacctgc tggagaacct 720
   gcaggcctac cagaagcgca tgggcgtgca gatgcagcgc ttcaagtgaa ctagt      775
<210> 4
   <211> 844
   <212> DNA
   <213> Artificial Sequence
<220>
   <223> Description of Artificial Sequence: Influenza-Matrix: Gen für sekretierte Form (mit N-terminaler Signalsequenz) mit erhöhtem G/C-Gehalt
<220>
   <223> Startcodon: atg (Nucleotide 11 bis 13), Stopcodon: tga (Nucleotide 836 bis 838)
<400> 4
   agatctaaag atggccgtca tggccccccg caccctggtg ctgctgctga gcggcgccct 60
   ggccctgacc cagacctggg ctagcctgct gaccgaggtg gagacctacg tgctgagcat 120
   catccccagc ggccccctga aggccgagat cgcccagagg ctggaggacg tgttcgccgg 180
   caagaacacc gacctggagg tgctgatgga gtggctgaag accaggccca tcctgagccc 240
   cctgaccaag ggcatcctgg gcttcgtgtt caccctgacc gtgcccagcg agcgcggcct 300
   gcagcgccgc cgcttcgtgc agaacgccct gaacggcaac ggcgacccca acaacatgga 360
   caaggccgtg aagctgtaca ggaagctgaa gagggagatc accttccacg gcgccaagga 420
   gatcagcctg agctacagcg ccggcgccct ggccagctgc atgggcctga tctacaacag 480
   gatgggcgcc gtgaccaccg aggtggcctt cggcctggtg tgcgccacct gcgagcagat 540
   cgccgacagc cagcaccgca gccacaggca gatggtgacc accaccaacc ccctgatcag 600
   gcacgagaac aggatggtgc tggccagcac caccgccaag gccatggagc agatggccgg 660
   cagcagcgag caggccgccg aggccatgga ggtggccagc caggccaggc agatggtgca 720
   ggccatgagg accatcggca cccaccccag cagcagcgcc ggcctgaaga acgacctgct 780
   ggagaacctg caggcctacc agaagcgcat gggcgtgcag atgcagcgct tcaagtgaac 840
   tagt                                                              844
<210> 5
   <211> 942
   <212> RNA
   <213> Artificial Sequence
<220>
   <223> Description of Artificial Sequence: Influenza-Matrix: mRNA mit Stabilisierungssequenzen
<220>
   <223> Die Stabilisierungssequenzen stammen aus den 5'- bzw. 3'-UTRs der β-Globin-mRNA von Xenopus laevis
<220>
   <223> Startcodon: aug (Nucleotide 56 bis 58), Stopcodon: uga (Nucleotide 812 bis 814)
<400> 5
   gcuuguucuu uuugcagaag cucagaauaa acgcucaacu uuggcagauc uaaagaugag 60
   ucuucuaacc gaggucgaaa cguacguucu cucuaucauc ccgucaggcc cccucaaagc 120
   cgagaucgca cagagacuug aagaugucuu ugcagggaag aacaccgauc uugagguucu 180
   cauggaaugg cuaaagacaa gaccaauccu gucaccucug acuaagggga uuuuaggauu 240
   uguguucacg cucaccgugc ccagugagcg aggacugcag cguagacgcu uuguccaaaa 300
   ugcccuuaau gggaacgggg auccaaauaa cauggacaaa gcaguuaaac uguauaggaa 360
   gcucaagagg gagauaacau uccauggggc caaagaaauc ucacucaguu auucugcugg 420
   ugcacuugcc aguuguaugg gccucauaua caacaggaug ggggcuguga ccacugaagu 480
   ggcauuuggc cugguaugug caaccuguga acagauugcu gacucccagc aucggucuca 540
   uaggcaaaug gugacaacaa ccaacccacu aaucagacau gagaacagaa ugguuuuagc 600
   cagcacuaca gcuaaggcua uggagcaaau ggcuggaucg agugagcaag cagcagaggc 660
   cauggagguu gcuagucagg cuaggcaaau ggugcaagcg augagaacca uugggacuca 720
   uccuagcucc agugcugguc ugaaaaauga ucuucuugaa aauuugcagg ccuaucagaa 780
   acgaaugggg gugcagaugc aacgguucaa gugaacuagu gacugacuag cccgcugggc 840
   cucccaacgg gcccuccucc ccuccuugca ccaaaaaaaa aaaaaaaaaa aaaaaaaaaa 900
   aaaaaaaaaa aaaaaaaaaa aaaaaaaaaa aaaaaaaaaa aa                    942
<210> 6
   <211> 942
   <212> RNA
   <213> Artificial Sequence
<220>
   <223> Description of Artificial Sequence: Influenza-Matrix: mRNA mit erhöhtem G/C-Gehalt und Stabilisierungssequenzen
<220>
   <223> Die Stabilisierungssequenzen stammen aus den 5'- bzw. 3'-UTRs der β-Globin-mRNA von Xenopus laevis
<220>
   <223> Startcodon: aug (Nucleotide 56 bis 58), Stopcodon: uga (Nucleotide 812 bis 814)
<400> 6
   gcuuguucuu uuugcagaag cucagaauaa acgcucaacu uuggcagauc uaaagaugag 60
   ccugcugacc gagguggaga ccuacgugcu gagcaucauc cccagcggcc cccugaaggc 120
   cgagaucgcc cagaggcugg aggacguguu cgccggcaag aacaccgacc uggaggugcu 180
   gauggagugg cugaagacca ggcccauccu gagcccccug accaagggca uccugggcuu 240
   cguguucacc cugaccgugc ccagcgagcg cggccugcag cgccgccgcu ucgugcagaa 300
   cgcccugaac ggcaacggcg accccaacaa cauggacaag gccgugaagc uguacaggaa 360
   gcugaagagg gagaucaccu uccacggcgc caaggagauc agccugagcu acagcgccgg 420
   cgcccuggcc agcugcaugg gccugaucua caacaggaug ggcgccguga ccaccgaggu 480
   ggccuucggc cuggugugcg ccaccugcga gcagaucgcc gacagccagc accgcagcca 540
   caggcagaug gugaccacca ccaacccccu gaucaggcac gagaacagga uggugcuggc 600
   cagcaccacc gccaaggcca uggagcagau ggccggcagc agcgagcagg ccgccgaggc 660
   cauggaggug gccagccagg ccaggcagau ggugcaggcc augaggacca ucggcaccca 720
   ccccagcagc agcgccggcc ugaagaacga ccugcuggag aaccugcagg ccuaccagaa 780
   gcgcaugggc gugcagaugc agcgcuucaa gugaacuagu gacugacuag cccgcugggc 840
   cucccaacgg gcccuccucc ccuccuugca ccaaaaaaaa aaaaaaaaaa aaaaaaaaaa 900
   aaaaaaaaaa aaaaaaaaaa aaaaaaaaaa aaaaaaaaaa aa                    942
<210> 7
   <211> 1011
   <212> RNA
   <213> Artificial Sequence
<220>
   <223> Description of Artificial Sequence: Influenza-Matrix: für sekretierte Form codierende mRNA mit erhöhtem G/C-Gehalt und Stabilisierungssequenzen
<220>
   <223> Startcodon: aug (Nucleotide 56 bis 58), Stopcodon: uga (Nucleotide 881 bis 883)
<400> 7
   gcuuguucuu uuugcagaag cucagaauaa acgcucaacu uuggcagauc uaaagauggc 60
   cgucauggcc ccccgcaccc uggugcugcu gcugagcggc gcccuggccc ugacccagac 120
   cugggccagc cugcugaccg agguggagac cuacgugcug agcaucaucc ccagcggccc 180
   ccugaaggcc gagaucgccc agaggcugga ggacguguuc gccggcaaga acaccgaccu 240
   ggaggugcug auggaguggc ugaagaccag gcccauccug agcccccuga ccaagggcau 300
   ccugggcuuc guguucaccc ugaccgugcc cagcgagcgc ggccugcagc gccgccgcuu 360
   cgugcagaac gcccugaacg gcaacggcga ccccaacaac auggacaagg ccgugaagcu 420
   guacaggaag cugaagaggg agaucaccuu ccacggcgcc aaggagauca gccugagcua 480
   cagcgccggc gcccuggcca gcugcauggg ccugaucuac aacaggaugg gcgccgugac 540
   caccgaggug gccuucggcc uggugugcgc caccugcgag cagaucgccg acagccagca 600
   ccgcagccac aggcagaugg ugaccaccac caacccccug aucaggcacg agaacaggau 660
   ggugcuggcc agcaccaccg ccaaggccau ggagcagaug gccggcagca gcgagcaggc 720
   cgccgaggcc auggaggugg ccagccaggc caggcagaug gugcaggcca ugaggaccau 780
   cggcacccac cccagcagca gcgccggccu gaagaacgac cugcuggaga accugcaggc 840
   cuaccagaag cgcaugggcg ugcagaugca gcgcuucaag ugaacuagug acugacuagc 900
   ccgcugggcc ucccaacggg cccuccuccc cuccuugcac caaaaaaaaa aaaaaaaaaa 960
   aaaaaaaaaa aaaaaaaaaa aaaaaaaaaa aaaaaaaaaa aaaaaaaaaa a          1011
<210> 8
   <211> 940
   <212> DNA
   <213> Homo sapiens
<220>
   <223> MAGE1: Wildtyp-Gen (zum Vergleich)
<220>
   <223> Startcodon: atg (Nucleotide 5 bis 7), Stopcodon: tga (Nucleotide 932 bis 934)
<400> 8
   catcatgtct cttgagcaga ggagtctgca ctgcaagcct gaggaagccc ttgaggccca 60
   acaagaggcc ctgggcctgg tgtgtgtgca ggctgccacc tcctcctcct ctcctctggt 120
   cctgggcacc ctggaggagg tgcccactgc tgggtcaaca gatcctcccc agagtcctca 180
   gggagcctcc gcctttccca ctaccatcaa cttcactcga cagaggcaac ccagtgaggg 240
   ttccagcagc cgtgaagagg aggggccaag cacctcttgt atcctggagt ccttgttccg 300
   agcagtaatc actaagaagg tggctgattt ggttggtttt ctgctcctca aatatcgagc 360
   cagggagcca gtcacaaagg cagaaatgct ggagagtgtc atcaaaaatt acaagcactg 420
   ttttcctgag atcttcggca aagcctctga gtccttgcag ctggtctttg gcattgacgt 480
   gaaggaagca gaccccaccg gccactccta tgtccttgtc acctgcctag gtctctccta 540
   tgatggcctg ctgggtgata atcagatcat gcccaagaca ggcttcctga taattgtcct 600
   ggtcatgatt gcaatggagg gcggccatgc tcctgaggag gaaatctggg aggagctgag 660
   tgtgatggag gtgtatgatg ggagggagca cagtgcctat ggggagccca ggaagctgct 720
   cacccaagat ttggtgcagg aaaagtacct ggagtaccgg caggtgccgg acagtgatcc 780
   cgcacgctat gagttcctgt ggggtccaag ggccctcgct gaaaccagct atgtgaaagt 840
   ccttgagtat gtgatcaagg tcagtgcaag agttcgcttt ttcttcccat ccctgcgtga 900
   agcagctttg agagaggagg aagagggagt ctgagcatga                       940
<210> 9
   <211> 308
   <212> PRT
   <213> Homo sapiens
<220>
   <223> Tumorantigen MAGE1: Proteinsequenz
<400> 9
   Ser Leu Glu Gln Arg Ser Leu His Cys Lys Pro Glu Glu Ala Leu Glu
   1 5 10 15
   Ala Gln Gln Glu Ala Leu Gly Leu Val Cys Val Gln Ala Ala Thr Ser
   20 25 30
   Ser Ser Ser Pro Leu Val Leu Gly Thr Leu Glu Glu Val Pro Thr Ala
   35 40 45
   Gly Ser Thr Asp Pro Pro Gln Ser Pro Gln Gly Ala Ser Ala Phe Pro
   50 55 60
   Thr Thr Ile Asn Phe Thr Arg Gln Arg Gln Pro Ser Glu Gly Ser Ser
   65 70 75 80
   Ser Arg Glu Glu Glu Gly Pro Ser Thr Ser Cys Ile Leu Glu Ser Leu
   85 90 95
   Phe Arg Ala Val Ile Thr Lys Lys Val Ala Asp Leu Val Gly Phe Leu
   100 105 110
   Leu Leu Lys Tyr Arg Ala Arg Glu Pro Val Thr Lys Ala Glu Met Leu
   115 120 125
   Glu Ser Val Ile Lys Asn Tyr Lys His Cys Phe Pro Glu Ile Phe Gly
   130 135 140
   Lys Ala Ser Glu Ser Leu Gln Leu Val Phe Gly Ile Asp Val Lys Glu
   145 150 155 160
   Ala Asp Pro Thr Gly His Ser Tyr Val Leu Val Thr Cys Leu Gly Leu
   165 170 175
   Ser Tyr Asp Gly Leu Leu Gly Asp Asn Gln Ile Met Pro Lys Thr Gly
   180 185 190
   Phe Leu Ile Ile Val Leu Val Met Ile Ala Met Glu Gly Gly His Ala
   195 200 205
   Pro Glu Glu Glu Ile Trp Glu Glu Leu Ser Val Met Glu Val Tyr Asp
   210 215 220
   Gly Arg Glu His Ser Ala Tyr Gly Glu Pro Arg Lys Leu Leu Thr Gln
   225 230 235 240
   Asp Leu Val Gln Glu Lys Tyr Leu Glu Tyr Arg Gln Val Pro Asp Ser
   245 250 255
   Asp Pro Ala Arg Tyr Glu Phe Leu Trp Gly Pro Arg Ala Leu Ala Glu
   260 265 270
   Thr Ser Tyr Val Lys Val Leu Glu Tyr Val Ile Lys Val Ser Ala Arg
   275 280 285
   Val Arg Phe Phe Phe Pro Ser Leu Arg Glu Ala Ala Leu Arg Glu Glu
   290 295 300
   Glu Glu Gly Val
   305
<210> 10
   <211> 939
   <212> RNA
   <213> Artificial Sequence
<220>
   <223> Description of Artificial Sequence: MAGE1: mRNA mit erhöhtem G/C-Gehalt
<220>
   <223> Startcodon: aug (Nucleotide 1 bis 3), Stopcodon: uga (Nucleotide 937 bis 939)
<400> 10
   augagccugg agcagcgcag ccugcacugc aagccggagg aggcgcugga ggcgcagcag 60
   gaggcgcugg gccuggucug cguccaggcg gcgacgagca gcagcagccc gcugguccug 120
   ggcacgcugg aggagguccc gacggcgggc agcacggacc cgccgcagag cccgcagggc 180
   gcgagcgcgu ucccgacgac gaucaacuuc acgcgccagc gccagccgag cgagggcagc 240
   agcagccgcg aggaggaggg cccgagcacg agcugcaucc uggagagccu guuccgcgcg 300
   gucaucacga agaaggucgc ggaccugguc ggcuuccugc ugcugaagua ccgcgcgcgc 360
   gagccgguca cgaaggcgga gaugcuggag agcgucauca agaacuacaa gcacugcuuc 420
   ccggagaucu ucggcaaggc gagcgagagc cugcagcugg ucuucggcau cgacgucaag 480
   gaggcggacc cgacgggcca cagcuacguc cuggucacgu gccugggccu gagcuacgac 540
   ggccugcugg gcgacaacca gaucaugccg aagacgggcu uccugaucau cguccugguc 600
   augaucgcga uggagggcgg ccacgcgccg gaggaggaga ucugggagga gcugagcguc 660
   auggaggucu acgacggccg cgagcacagc gcguacggcg agccgcgcaa gcugcugacg 720
   caggaccugg uccaggagaa guaccuggag uaccgccagg ucccggacag cgacccggcg 780
   cgcuacgagu uccugugggg cccgcgcgcg cuggcggaga cgagcuacgu caagguccug 840
   gaguacguca ucaaggucag cgcgcgcguc cgcuucuucu ucccgagccu gcgcgaggcg 900
   gcgcugcgcg aggaggagga gggcgucuga gcgugauga 939
<210> 11
   <211> 939
   <212> RNA
   <213> Artificial Sequence
<220>
   <223> Description of Artificial Sequence: MAGE1: mRNA mit alternativer Codonverwendung
<220>
   <223> Startcodon: aug (Nucleotide 1 bis 3), Stopcodon: uga (Nucleotide 937 bis 939)
<400> 11
   augagccugg agcagcgcag ccugcacugc aagcccgagg aggcccugga ggcccagcag 60
   gaggcccugg gccuggugug cgugcaggcc gccaccagca gcagcagccc ccuggugcug 120
   ggcacccugg aggaggugcc caccgccggc agcaccgacc ccccccagag cccccagggc 180
   gccagcgccu uccccaccac caucaacuuc acccgccagc gccagcccag cgagggcagc 240
   agcagccgcg aggaggaggg ccccagcacc agcugcaucc uggagagccu guuccgcgcc 300
   gugaucacca agaagguggc cgaccuggug ggcuuccugc ugcugaagua ccgcgcccgc 360
   gagcccguga ccaaggccga gaugcuggag agcgugauca agaacuacaa gcacugcuuc 420
   cccgagaucu ucggcaaggc cagcgagagc cugcagcugg uguucggcau cgacgugaag 480
   gaggccgacc ccaccggcca cagcuacgug cuggugaccu gccugggccu gagcuacgac 540
   ggccugcugg gcgacaacca gaucaugccc aagaccggcu uccugaucau cgugcuggug 600
   augaucgcca uggagggcgg ccacgccccc gaggaggaga ucugggagga gcugagcgug 660
   auggaggugu acgacggccg cgagcacagc gccuacggcg agccccgcaa gcugcugacc 720
   caggaccugg ugcaggagaa guaccuggag uaccgccagg ugcccgacag cgaccccgcc 780
   cgcuacgagu uccugugggg cccccgcgcc cuggccgaga ccagcuacgu gaaggugcug 840
   gaguacguga ucaaggugag cgcccgcgug cgcuucuucu uccccagccu gcgcgaggcc 900
   gcccugcgcg aggaggagga gggcguguga gccugauga 939
<210> 12
   <211> 7
   <212> RNA
   <213> Artificial Sequence
<220>
   <223> Description of Artificial Sequence: Ein für eine Endonuklease erkennbares Sequenzmotiv, das im 3'UTR-Segment des für den Transferin-Rezeptor codierenden Gens enthalten ist (S. 10 der Beschreibung).
<400> 12
   gaacaag 7
<210> 13
   <211> 13
   <212> RNA
   <213> Artificial Sequence
<220>
   <223> Description of Artificial Sequence: Kozak-Sequenz, Ribosomen-Bindungsstelle (S. 12 der Beschreibung)
<400> 13
   gccgccacca ugg 13

## Beschreibung

Die vorliegende Erfindung betrifft eine pharmazeutische Zusammensetzung, enthaltend eine mRNA, die durch Sequenzmodifikationen im translatierten Bereich stabilisiert und für die Translation optimiert ist bzw. die entsprechende modifizierte mRNA. Die erfindungsgemäße pharmazeutische Zusammensetzung eignet sich als Therapeutikum zur Gentherapie insbesondere zur Geweberegeneration.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide.

Die übliche Vorgehensweise bisheriger Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, wie bspw. Calciumphosphat-Transfektion, Polypren-Transfektion, Protoplasten-Fusion, Elektroporation, Mikroinjektion und Lipofektion, entwickelt worden, wobei sich insbesondere die Lipofektion als geeignetes Verfahren herausgestellt hat.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, daß aufgrund ihrer infektiösen Eigenschaften eine sehr hohe Transfektionsrate zu erzielen ist. Die verwendeten Viren werden genetisch verändert, so daß in der transfizierten Zelle keine funktionsfähigen infektiösen Partikel gebildet werden. Trotz dieser Vorsichtsmaßnahme kann jedoch aufgrund möglicher Rekombinationsereignisse ein gewisses Risiko der unkontrollierten Ausbreitung der eingebrachten gentherapeutisch wirksamen sowie viralen Gene nicht ausgeschlossen werden.

Üblicherweise wird die in die Zelle eingebrachte DNA in gewissem Ausmaß in das Genom der transfizierten Zelle integriert. Einerseits kann dieses Phänomen einen erwünschten Effekt ausüben, da hierdurch eine langandauernde Wirkung der eingebrachten DNA erzielt werden kann. Andererseits bringt die Integration in das Genom ein wesentliches Risiko der Gentherapie mit sich. So kann es bspw. zu einer Insertion der eingebrachten DNA in ein intaktes Gen kommen, was eine Mutation darstellt, welche die Funktion des endogenen Gens behindert oder gar vollkommen ausschaltet. Durch solche Integrationsereignisse können einerseits für die Zelle lebenswichtige Enzymsysteme ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidendes Gen verändert wird. Daher kann bei der Verwendung von DNA-Viren als Gentherapeutika und Vakzine ein Risiko der Krebsbildung nicht ausgeschlossen werden. In diesem Zusammenhang ist auch zu beachten, daß zur wirksamen Expression der in die Zelle eingebrachten Gene die entsprechenden DNA-Vehikel einen starken Promotor, bspw. den viralen CMV-Promotor, enthalten. Die Integration derartiger Promotoren in das Genom der behandelten Zelle kann zu unerwünschten Veränderungen der Regulierung der Genexpression in der Zelle führen.

Ein weiterer Nachteil der Verwendung von DNA als Gentherapeutika und Vakzine ist die Induktion pathogener Anti-DNA-Antikörper im Patienten unter Hervorrufung einer möglicherweise tödlichen Immunantwort.

Im Gegensatz zu DNA ist der Einsatz von RNA als Gentherapeutikum oder Vakzin als wesentlich sicherer einzustufen. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden. Daher kann RNA für molekularmedizinische Therapieverfahren als Molekül der Wahl angesehen werden.

Allerdings bedürfen auf RNA-Expressionssystemen beruhende medizinische Verfahren vor einer breiteren Anwendung noch der Lösung einiger grundsätzlicher Probleme. Eines der Probleme bei der Verwendung von RNA ist der schere, Zell- bzw. Gewebespezifische effiziente Transfer der Nukleinsäure. Da sich RNA in Lösung normalerweise als sehr instabil erweist, konnte durch die herkömmlichen Verfahren, die bei DNA verwendet werden, RNA bisher nicht oder nur sehr ineffizient als Therapeutikum bzw. Impfstoff verwendet werden.

Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Der natürliche Abbau von mRNA im Cytoplasma von Zellen ist sehr fein reguliert. Diesbezüglich sind mehrere Mechanismen bekannt. So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Annu. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Im Stand der Technik sind einige Maßnahmen vorgeschlagen worden, um die Stabilität von RNA zu erhöhen und dadurch ihren Einsatz als Gentherapeutikum bzw. RNA-Vakzine zu ermöglichen.

In diesem Zusammenhang beschreibt WO 98/34640 jedoch nur synthetische DNA Moleküle, die HIV gag und Modifikationen von HIV gag kodieren. Die in WO 98/34640 offenbarten Moleküle können als Polynukleotid-Vakzine eingesetzt werden, die eine effektive Immunprophylaxe gegen HIV durch Stimulierung neutralisierender Antikörper und Zellvermittelter Immunität ermöglichen. WO 98/34640 beschreibt in diesem Zusammenhang jedoch keine Erhöhungen oder gar Maximierungen des G/C-Gehaltes der kodierenden synthetischen DNA Moleküle und ebenfalls keine in diesem Zusammenhang verwendeten und modifizierten mRNA-Moleküle.

Auch WO 97/48370 offenbart zwar synthetische DNA-Moleküle, die ein Peptid oder Protein kodieren, insbesondere HIV env als auch Modifikationen on HIV env. WO 97/48370 beschreibt aber auch keinerlei Erhöhungen oder gar Maximierungen des G/C-Gehaltes der kodierenden synthetischen DNA Moleküle und ebenfalls keine Modifikationen der in diesem Zusammenhang verwendeten mRNA-Moleküle.

In EP-A-1083232 wird zur Lösung des vorstehend genannten Problems der Instabilität von RNA *ex vivo* ein Verfahren zur Einbringung von RNA, insbesondere mRNA, in Zellen und Organismen vorgeschlagen, bei welchem die RNA in Form eines Komplexes mit einem kationischen Peptid oder Protein vorliegt. So beschreibt die EP 1083232 auch die Verwendung derartiger Komplexe zur Behandlung von Erkrankungen, die auf mindestens einem genetischen Defekt beruhen. Hierbei wird durch die komplexierte mRNA die korrekte genetische Information bereitgestellt, die im fehlerhaften oder abwesenden Gen des Patienten nicht entsprechend vorliegt.

WO 99/14346 beschreibt weitere Verfahren zur Stabilisierung von mRNA. Insbesondere werden Modifizierungen der mRNA vorgeschlagen, welche die mRNA-Spezies gegenüber dem Abbau von RNasen stabilisieren. Derartige Modifikationen betreffen einerseits die Stabilisierung durch Sequenzmodifikationen, insbesondere Verminderung des C- und/oder U-Gehalts durch Baseneliminierung oder Basensubstitution. Andererseits werden chemische Modifikationen, insbesondere die Verwendung von Nucleotidanaloga, sowie 5'- und 3'- Blockierungsgruppen, eine erhöhte Länge des Poly-A-Schwanzes sowie die Komplexierung der mRNA mit stabilisierenden Mitteln und Kombinationen der genannten Maßnahmen vorgeschlagen.

In den US-Patenten US 5,580,859 und US 6,214,804 werden unter anderem im Rahmen der "transienten Gentherapie" (TGT) mRNA-Vakzine und -Therapeutika offenbart. Es werden verschiedene Maßnahmen zur Erhöhung der Translationseffizienz und der mRNA-Stabilität beschrieben, die sich vor allem auf die nicht-translatierten Sequenzbereiche beziehen.

Bieler und Wagner (in: Schleef (Hrsg.), Plasmids for Therapy and Vaccination, Kapitel 9, Seiten 147 bis 168, Wiley-VCH, Weinheim, 2001) berichten von der Verwendung synthetischer Gene im Zusammenhang mit gentherapeutischen Methoden unter Verwendung von DNA-Vakzinen und lentiveralen Vektoren. Es wird die Konstruktion eines synthetischen, von HIV-1 abgeleiteten *gag*-Gens beschrieben, bei welchem die Codons gegenüber der Wildtyp-Sequenz derart modifiziert wurden (alternative Codonverwendung, engl. "codon usage"), daß sie der Verwendung von Codons entsprach, die in hoch exprimierten Säugergenen zu finden ist. Dadurch wurde insbesondere der A/T-Gehalt gegenüber der Wildtyp-Sequenz vermindert. Die Autoren stellen insbesondere eine erhöhte Expressionsrate des synthetischen *gag*-Gens in transfizierten Zellen fest. Des weiteren wurde in Mäusen eine erhöhte Antikörperbildung gegen das *gag*-Protein bei mit dem synthetischen DNA-Konstrukt immunisierten Mäusen und auch eine verstärkte Cytokinfreisetzung *in vitro* bei transfizierten Milzzellen von Mäusen beobachtet. Schließlich konnte eine Induzierung einer cytotoxischen Immunantwort in mit dem *gag*-Expressionsplasmid immunisierten Mäusen festgestellt werden. Die Autoren dieses Artikels führen die verbesserten Eigenschaften ihres DNA-Vakzins im wesentlichen auf einen durch die optimierte Codonverwendung hervorgerufene Veränderung des nucleo-cytoplasmatischen Transports der vom DNA-Vakzin exprimierten mRNA zurück. Im Gegensatz dazu halten die Autoren die Auswirkung der veränderten Codonverwendung auf die Translationseffizienz für gering.

Der vorliegenden Erfindung liegt daher die Aufgabe zugrunde, ein neues System zur Gentherapie bereitzustellen, das die mit den Eigenschaften von DNA-Therapeutika verbundenen Nachteile überwindet und die Wirksamkeit von auf RNA-Spezies basierenden Therapeutika erhöht.

Diese Aufgabe wird durch die in den Ansprüchen gekennzeichneten Ausführungsformen der vorliegenden Erfindung gelöst.

Insbesondere wird erfindungsgemäß eine modifizierte mRNA sowie mindestens eine derartige modifizierte mRNA in Verbindung mit einem pharmazeutisch, auch zur Herstellung eines Medikaments zur Gentherapie, verträglichen Träger und/oder Vehikel enthaltende pharmazeutische Zusammensetzung bereitgestellt, wobei die modifizierte mRNA für mindestens ein biologisch wirksames Peptid oder Polypeptid codiert, welches in dem zu behandelnden Patienten nicht nur unzureichend oder fehlerhalft gebildet wird, wobei die Sequenz der mRNA, insbesondere im für das mindestens eine Peptid oder Polypeptid codierenden Bereich, gegenüber der Wildtyp-mRNA folgende Modifikation aufweist.

Der G/C-Gehalt des für das Peptid oder Polypeptid codierenden Bereichs der modifizierten erfindungsgemäßen mRNA ist größer als der G/C-Gehalt des codierenden Bereichs der für das Peptid oder Polypeptid codierenden Wildtyp-mRNA, wobei die codierte Aminosäuresequenz gegenüber dem Wildtyp unverändert ist.

Diese Modifikation beruht auf der Tatsache, daß für eine effiziente Translation einer mRNA die Sequenzabfolge des zu translatierenden Bereichs der mRNA wesentlich ist. Dabei spielt die Zusammensetzung und die Abfolge der verschiedenen Nucleotide eine große Rolle. Insbesondere sind Sequenzen mit erhöhtem G(Guanosin)/C(Cytosin)-Gehalt stabiler als Sequenzen mit einem erhöhten A(Adenosin)/U(Uracil)-Gehalt. Daher werden erfindungsgemäß unter Beibehaltung der translatierten Aminosäureabfolge die Codons gegenüber der Wildtyp-mRNA derart variiert, daß sie vermehrt G/C-Nucleotide beinhalten. Da mehrere Codons für ein und dieselbe Aminosäure codieren (Degeneration des genetischen Codes), können die für die Stabilität günstigsten Codons ermittelt werden (alternative Codonverwendung, engl. "codon usage").

In Abhängigkeit von der durch die modifizierte erfindungsgemäße mRNA zu codierenden Aminosäure sind unterschiedliche Möglichkeiten zur Modifikation der mRNA-Sequenz gegenüber der Wildtyp-Sequenz möglich. Im Fall von Aminosäuren, die durch Codons codiert werden, die ausschließlich G- oder C- Nucleotide enthalten, ist keine Modifikation des Codons erforderlich. So erfordern die Codons für Pro (CCC oder CCG), Arg (CGC oder CGG), Ala (GCC oder GCG) und Gly (GGC oder GGG) keine Veränderung, da kein A oder U vorhanden ist.

In folgenden Fällen werden die Codons, welche A-und/oder U-Nucleotide enthalten durch Substituieren anderer Codons, welche die gleichen Aminosäuren codieren, jedoch kein A und/oder U enthalten, verändert. Beispiele sind:
Die Codons für Pro können von CCU oder CCA zu CCC oder CCG verändert werden;
die Codons für Arg können von CGU oder CGA oder AGA oder AGG zu CGC oder CGG verändert werden;
die Codons für Ala können von GCU oder GCA zu GCC oder GCG verändert werden;
die Codons für Gly können von GGU oder GGA zu GGC oder GGG verändert werden.

In anderen Fällen können A bzw. U-Nucleotide zwar nicht aus den Codons eliminiert werden, es ist jedoch möglich, den A- und U-Gehalt durch Verwendung von Codons zu vermindern, die weniger A- und/oder U-Nucleotide enthalten. Zum Beispiel:
Die Codons für Phe können von UUU zu UUC verändert werden;
die Codons für Leu können von UUA, CUU oder CUA zu CUC oder CUG verändert werden;
die Codons für Ser können von UCU oder UCA oder AGU zu UCC, UCG oder AGC verändert werden;
das Codon für Tyr kann von UAU zu UAC verändert werden;
das Stop-Codon UAA kann zu UAG oder UGA verändert werden;
das Codon für Cys kann von UGU zu UGC verändert werden;
das Codon His kann von CAU zu CAC verändert werden;
das Codon für Gln kann von CAA zu CAG verändert werden;
die Codons für Ile können von AUU oder AUA zu AUC verändert werden;
die Codons für Thr können von ACU oder ACA zu ACC oder ACG verändert werden;
das Codon für Asn kann von AAU zu AAC verändert werden;
das Codon für Lys kann von AAA zu AAG verändert werden;
die Codons für Val können von GUU oder GUA zu GUC oder GUG verändert werden;
das Codon für Asp kann von GAU zu GAC verändert werden;
das Codon für Glu kann von GAA zu GAG verändert werden.

Im Falle der Codons für Met (AUG) und Trp (UGG) besteht hingegen keine Möglichkeit der Sequenzmodifikation.

Die vorstehend aufgeführten Substitutionen können selbstverständlich einzeln aber auch in allen möglichen Kombinationen zur Erhöhung des G/C-Gehalts der modifizierten erfindungsgemäßen mRNA gegenüber der ursprünglichen Sequenz verwendet werden. So können beispielsweise alle in der ursprünglichen (Wildtyp-) Sequenz auftretenden Codons für Thr zu ACC (oder ACG) verändert werden. Vorzugsweise werden jedoch Kombinationen der vorstehenden Substitutionsmöglichkeiten genutzt, z.B.:
Substitution aller in der ursprünglichen Sequenz für Thr codierenden Codons zu ACC (oder ACG) und Substitution aller ursprünglich für Ser codierenden Codons zu UCC ( oder UCG oder AGC);
Substitution aller in der ursprünglichen Sequenz für Ile codierenden Codons zu AUC und Substitution aller ursprünglich für Lys codierenden Codons zu AAG und Substitution aller ursprünglich für Tyr codierenden Codons zu UAC;
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Arg codierenden Codons zu CGC (oder CGG);
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Gly codierenden Codons zu GGC (oder GGG) und Substituion aller ursprünglich für Asn codierenden Codons zu AAC;
Substitution aller in der ursprünglichen Sequenz für Val codierenden Codons zu GUC (oder GUG) und Substitution aller usprünglich für Phe codierenden Codons zu UUC und Substitution aller ursprünglich für Cys codierenden Codons zu UGC und Substitution aller ursprünglich für Leu codierenden Codons zu CUG (oder CUC) und Substitution aller ursprünglich für Gln codierenden Codons zu CAG und Substitution aller ursprünglich für Pro codierenden Codons zu CCC (oder CCG);
usw.

Vorzugsweise wird der G/C-Gehalt des für das Peptid bzw. Polypeptid codierenden Bereichs der modifizierten erfindungsgemäßen mRNA um mindestens 7%-Punkte, mehr bevorzugt um mindestens 15%-Punkte, besonders bevorzugt um mindestens 20%-Punkte gegenüber dem G/C-Gehalt des codierten Bereichs der für das Polypeptid codierenden Wildtyp-mRNA erhöht.

Besonders bevorzugt ist es in diesem Zusammenhang, den G/C-Gehalt der modifizierten mRNA insbesondere zur Verwendung zur Herstellung eines Medikaments, insbesondere im für das mindestens eine Peptid bzw. Polypeptid codierenden Bereich, im Vergleich zur Wildtyp-Sequenz maximal zu erhöhen.

Erfindungsgemäß besonders bevorzugt ist es, den erfindungsgemäß in der modifizierten mRNA erhöhten, insbesondere maximalen, sequenziellen G/C-Anteil mit den "häufigen" Codons zu verknüpfen, ohne die Aminosäuresequenz des durch den codierenden Bereich der mRNA codierten Peptids bzw. Polypeptids (ein oder mehrere) zu verändern. Diese bevorzugte Ausführungsform stellt eine besonders effizient translatierte und stabilisierte mRNA bspw. für die erfindungsgemäße pharmazeutische Zusammensetzung bereit.

In den Sequenzen eukaryotischer mRNAs gibt es destabilisierende Sequenzelemente (DSE), an welche Signalproteine binden und den enzymatischen Abbau der mRNA *in vivo* regulieren. Daher werden zur weiteren Stabilisierung der in der erfindungsgemäßen pharmazeutischen Zusammensetzung enthaltenen modifizierten mRNA gegebenenfalls im für das mindestens eine Peptid bzw. Polypeptid codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es ebenfalls bevorzugt, gegebenenfalls in den nicht-translatierten Bereichen (3'- und/oder 5'-UTR) vorhandene DSE aus der erfindungsgemäßen mRNA zu eliminieren.

Derartige destabilisierende Sequenzen sind bspw. AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674). Die in der erfindungsgemäßen pharmazeutischen Zusammensetzung enthaltenen erfindungsgemäßen RNA-Moleküle sind daher vorzugsweise derart gegenüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Auch diese Sequenzmotive werden bevorzugt in der modifizierten mRNA der erfindungsgemäßen pharmazeutischen Zusammensetzung eliminiert.

Einem Fachmann sind verschiedene Verfahren geläufig, die zur Substitution von Codons in der erfindungsgemäßen modifizierten mRNA geeignet sind. Im Falle kürzerer codierender Bereiche (die für biologisch wirksame Peptide codieren) kann bspw. die gesamte mRNA chemisch unter Verwendung von Standardtechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt; Maniatis et al., Molecular Cloning: A Laboratory Manual, Cold Spring Harbor Laboratory Press, 3. Aufl., Cold Spring Harbor, NY, 2001.

Bei diesem Verfahren wird zur Herstellung der erfindungsgemäßen mRNA daher ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nucleotidsequenz für die herzustellende mRNA und ein Termisationssignal für die in *in vitro* Transkribtion folgen. Das DNA-Molekül, das die Matrize des herzustellenden RNA-Konstrukts bildet, kann durch fermentative Vermehrung und anschließende Isolierung als Teil eines in Bakterien replizierbaren Plasmids hergestellt werden. Als für die vorliegende Erfindung geeignete Plasmide können bspw. die Plasmide pT7Ts (GenBank-Zugriffsnummer U26404; Lai et al., Development 1995, 121: 2349 bis 2360), pGEM^{®}-Reihe, bspw. pGEM^{®}-1 (GenBank-Zugriffsnummer X65300; von Promega) und pSP64 (GenBank-Zugriffsnummer X65327) genannt werden; vgl. auch Mezei und Storts, Purification of PCR Products, in: Griffin und Griffin (Hrsg.), PCR Technology: Current Innovation, CRC Press, Boca Raton, FL, 2001.

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restriktionsendonukleasen ausgeschnitten.

Die erfindungsgemäß modifizierte mRNA, welche in der erfindungsgemäßen pharmazeutischen Zusammensetzung enthalten ist, und zur Herstellung eines Medikaments zur Gentherapie verwendet wird, kann darüber hinaus eine 5'-Cap-Struktur (ein modifiziertes Guanosin-Nucleotid) aufweisen. Als Beispiele von Cap-Strukturen können m7G(5')ppp (5'(A,G(5')ppp(5')A und G(5')ppp(5')G genannt werden.

Gemäß einer weiteren bevorzugten Ausführungsform der vorliegenden Erfindung enthält die erfindungsgemäß modifizierte mRNA einen PolyA-Schwanz von mindestens 50 Nuclotiden, vorzugsweise mindestens 70 Nucleotiden, mehr bevorzugt mindestens 100 Nucleotiden, besonders bevorzugt mindestens 200 Nucleotiden.

Für eine effiziente Translation der erfindungsgemäßen mRNA ist weiterhin eine wirksame Bindung der Ribosomen an die Ribosomen-Bindungsstelle (Kozak-Sequenz: GCCGCCACCAUGG, das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht.

Des weiteren ist es möglich, in die modifizierte und erfindungsgemäß mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische mRNA"). Beispiele erfindungsgemäß verwendbarer IRES-Sequenzen sind diejenigen aus Picomaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorliegenden Erfindung weist die modifizierte erfindungsgemäße mRNA in den 5'- und/oder 3'-nicht translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis*, genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für α-Globin, α-(I)-Collagen, 15-Lipoxygenase oder für Tyrosin-Hydroxylase codiert (vgl. Holcik et al., Proc. Natl. Acad. Sci. USA 1997, 94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Stabilisierung der modifizierten und erfindungsgemäß mRNA ist es außerdem bevorzugt, daß diese mindestens ein Analoges natürlich vorkommender Nucleotide aufweist. Dies beruht auf der Tatsache, daß die in den Zellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseffizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Erfindungsgemäß können derartige Analoga in nicht-translatierten und translatierten Bereichen der modifizierten erfindungsgemäßen mRNA vorkommen.

Des weiteren kann der wirksame Transfer der modifizierten und erfindungsgemäß mRNA in die zu behandelnden Zellen bzw. den zu behandelnden Organismus dadurch verbessert werden, daß die modifizierte mRNA mit einem kationischen Peptid oder Protein assoziiert oder daran gebunden ist. Insbesondere ist dabei die Verwendung von Protamin als polykationischem, Nucleinsäure-bindenden Protein besonders wirksam. Des weiteren ist die Verwendung anderer kationischer Peptide oder Proteine, wie Poly-L-Lysin oder Histonen, ebenfalls möglich. Diese Vorgehensweise zur Stabilisierung der modifizierten erfindungsgemäßen mRNA ist in EP-A-1083232 beschrieben.

Bei der gentherapeutischen Verwendung der erfindungsgemäßen pharmazeutischen Zusammensetzung (bzw. bei der Verwendung der mRNA zur Gentherapie bzw. bei der Verwendung der mRNA zur Herstelllung einer pharmazeutischen Zusammensetzung zur Gentherapie) codiert die modifizierte mRNA für mindestens ein biologisch wirksames Peptid oder Polypeptid, welches in dem zu behandelnden Patienten bspw. nicht, nur unzureichend oder fehlerhaft gebildet wird. Daher sind Beispiele von der erfindungsgemäßen mRNA codierte Polypeptide Dystrophin, der Chloridkanal, der bei der cystischen Fibrose fehlerhaft verändert ist, Enzyme, die bei metabolischen Erkrankungen, wie Phenylketonurie, Galktosämie, Homocystinurie, Adenosindeaminasedefizienz usw., fehlen bzw. fehlerhaft sind, Enzyme, die an der Synthese von Neurotransmittern, wie Dopamin, Norepinephrin und GABA beteiligt sind, insbesondere Tyrosinhydroxylase und DOPA-Decarboxylase, α-1-Antitrypsin usw. Des weiteren kann die pharmazeutische Zusammensetzung zur Abgabe von Zelloberflächenrezeptoren und Molekülen, die an derartige Rezeptoren binden, verwendet werden, indem die in der pharmazeutischen Zusammensetzung enthaltene modifizierte mRNA für ein derartiges biologisch wirksames Protein bzw. Peptid codiert. Beispiele derartiger Proteine, die extrazellulär wirken oder an Zelloberflächenrezeptoren binden, sind bspw. Gewebsplasminogenaktivator (TPA), Wachstumshormone, Insulin, Interferone, Granulocyten-Makrophagen-Koloniestimulisierungsfaktor (GM-CFS), Erythropoietin (EPO) usw. Durch Auswahl geeigneter Wachstumsfaktoren kann die pharmazeutische Zusammensetzung der vorliegenden Erfindung bspw. zur Geweberegeneration verwendet werden. Dadurch können Erkrankungen, die durch eine Gewebedegeneration gekennzeichnet sind, bspw. neurodegenerative Erkrankungen, wie Morbus Alzheimer, Morbus Parkinson usw., und andere degenerative Erkrankungen, bspw. Arthrose, behandelt werden. In diesen Fällen codiert die, insbesondere in der pharmazeutischen Zusammensetzung der vorliegenden Erfindung enthaltene, modifizierte mRNA vorzugsweise für Wachstumsfaktoren aus der TGF-β -Familie, wobei insbesondere EGF, FGF, PDGF, BMP, GDNF, BDNF, GDF und neurotrophe Faktoren, wie NGF, Neutrophine usw. zu nennen sind.

Darüber hinaus kann die erfindungsgemäß modifizierte mRNA neben dem gentherapeutisch wirksamen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-1, IL-2, IL-3, IL-4, IL-5, IL-6, IL-7, IL-8, IL-9, IL-10, IL-12, INF-α, INF-γ, GM-CFS, LT-α oder Wachstumsfaktoren, wie hGH, codiert.

Die erfindungsgemäße pharmazeutische Zusammensetzung enthält neben der modifizierten erfindungsgemäßen mRNA einen pharmazeutisch verträglichen Träger und/oder ein pharmazeutisch verträgliches Vehikel. Entsprechende Wege zur geeigneten Formulierung und Herstellung der erfindungsgemäßen pharmazeutischen Zusammensetzung sind bei "Remington's Pharmaceutical Sciences" (Mack Pub. Co., Easton, PA, 1980) offenbart, das vollinhaltlich Bestandteil der Offenbarung der vorliegenden Erfindung ist. Für die parenterale Verabreichung kommen als Trägerstoffe bspw. steriles Wasser, sterile Kochsalzlösungen, Polyalkylenglykole, hydrogenierte Naphthalen und insbesondere biokompatible Lactidpolymere, Lactid/Glycolidcopolymer oder Polyoxyethylen-/Polyoxypropylencopolymere in Betracht. Erfindungsgemäße Zusammensetzungen können Füllsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metallionen oder Einschluß von Materialien in oder auf besondere Präparationen von Polymerverbindung, wie z.B. Polylactat, Polyglykolsäure, Hydrogel oder auf Liposomen, Mikroemulsion, Micellen, unilamellare oder multilamellare Vesikel, Erythrozyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wirkstoffkomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Poloxamere oder Poloxamine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Citrat oder Acetat usw. verwendet wird, und der pH typischerweise auf 5,0 bis 8,0, vorzugsweise 6,0 bis 7,0, eingestellt wird. Der Träger bzw. das Vehikel - wird zusätzlich vorzugsweise Salzbestandteile enthalten, z.B. Natriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortschrittsstadium, wie auch dem Körpergewicht, dem Alter und dem Geschlecht des Patienten ab.

Die Konzentration der modifizierten erfindungsgemäßen mRNA in derartigen Formulierungen kann daher innerhalb eines weiten Bereichs von 1 µg bis 100 mg/ml variieren. Die erfindungsgemäße pharmazeutische Zusammensetzung wird vorzugsweise parenteral, bspw. intravenös, intraarteriell, subkutan, intramuskulär, dem Patienten verabreicht. Ebenso ist es möglich, die pharmazeutische Zusammensetzung topisch oder oral zu verabreichen.

Somit kann erfindungsgemäß auch ein Verfahren zur Behandlung der vorstehend genannten Krankheiten eingesetzt werden, welches das Verabreichen der erfindungsgemäßen pharmazeutischen Zusammensetzung an einen Patienten, insbesondere einen Menschen, umfasst.

Darüber hinaus kann ebenfalls ein Verfahren eingesetzt werden, das der Ermittlung der modifizierten Sequenz der in der erfindungsgemäßen pharmazeutischen Zusammensetzung enthaltenen mRNA dient. Dabei kann bspw. die Adaptierung der RNA-Sequenzen mit zwei unterschiedlichen Optimierungszielen durchgeführt werden. Zum einen mit größtmöglichen G/C-Gehalt, zum anderen ggf. unter bestmöglicher Berücksichtigung der Häufigkeit der tRNAs gemäß Codon Usage. Im ersten Schritt des Verfahrens wird eine virtuelle Translation einer beliebigen RNA(oder DNA)-Sequenz durchgeführt, um die entsprechende Aminosäuresequenz zu generieren. Ausgehend von der Aminosäuresequenz wird eine virtuelle reverse Translation durchgeführt, die aufgrund des degenerierten genetischen Codes Wahlmöglichkeiten für die entsprechenden Codons liefert. Je nach geforderter Optimierung bzw. Modifizierung werden zur Auswahl der geeigneten Codons entsprechende Selektionslisten und Optimierungsalgorithmen verwendet. Die Ausführung der Algorithmen erfolgt typischerweise mit Hilfe einer geeigneten Software auf einem Computer. So wird die optimierte mRNA-Sequenz erstellt und kann bspw. mit Hilfe einer entsprechenden Anzeigevorrichtung ausgegeben und mit der usrpünglichen (Wildtyp-)Sequenz verglichen werden. Das gleiche gilt auch für die Häufigkeit der einzelnen Nucleotide. Die Änderungen gegenüber der ursprünglichen Nucleotidsequenz werden dabei vorzugsweise hervorgehoben. Weiter werden gemäß einer bevorzugten Ausführungsform in der Natur bekannte stabile Sequenzen eingelesen, welche die Grundlage für eine gemäß natürlichen Sequenzmotiven stabilisierte RNA bieten können. Es kann ebenfalls eine Sekundärstrukturanalyse vorgesehen werden, die anhand von Strukturberechnungen stabilisierende und instabilisierende Eigenschaften bzw. Bereiche der RNA analysieren kann.

Die Figuren zeigen:

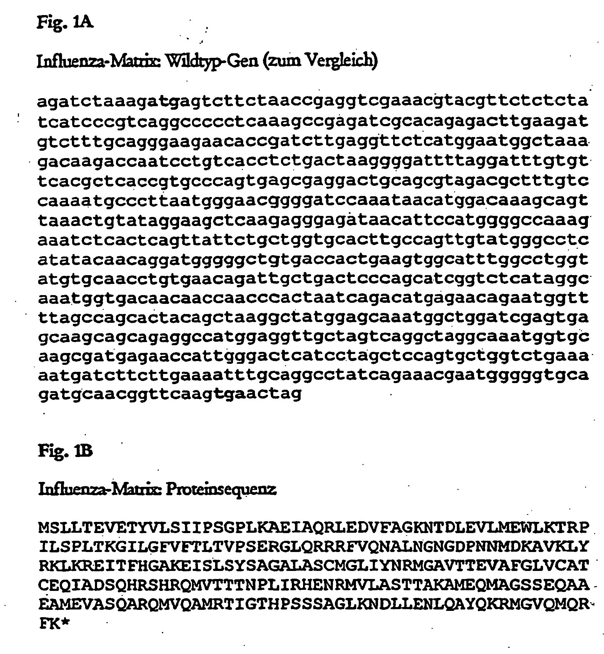

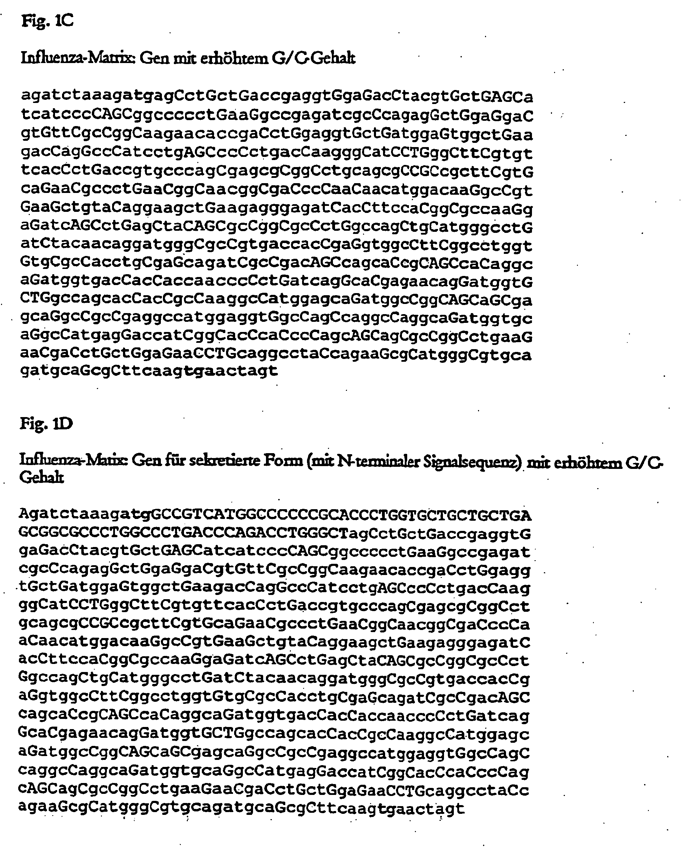

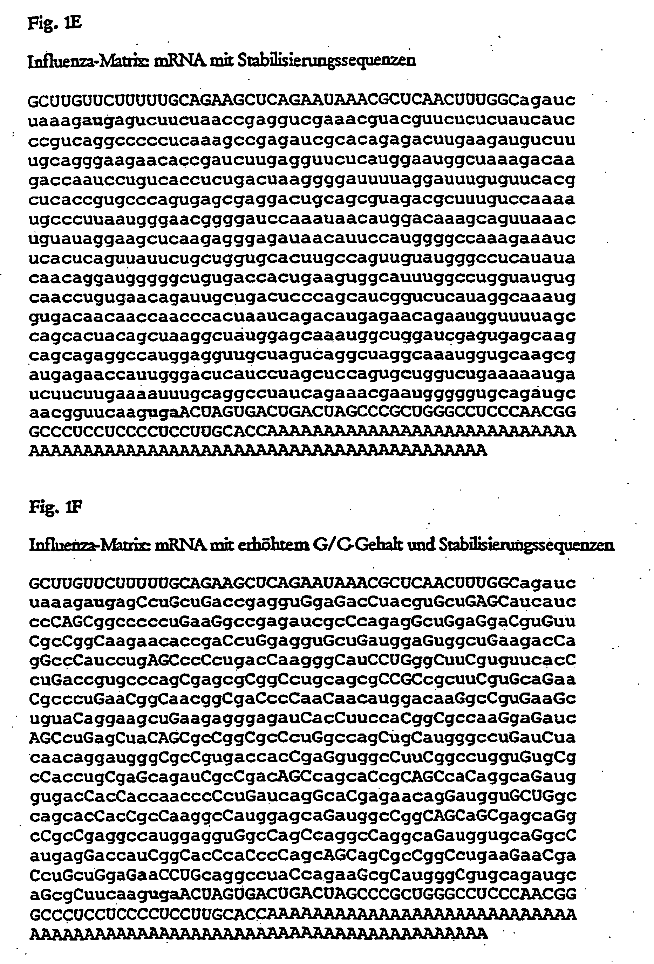

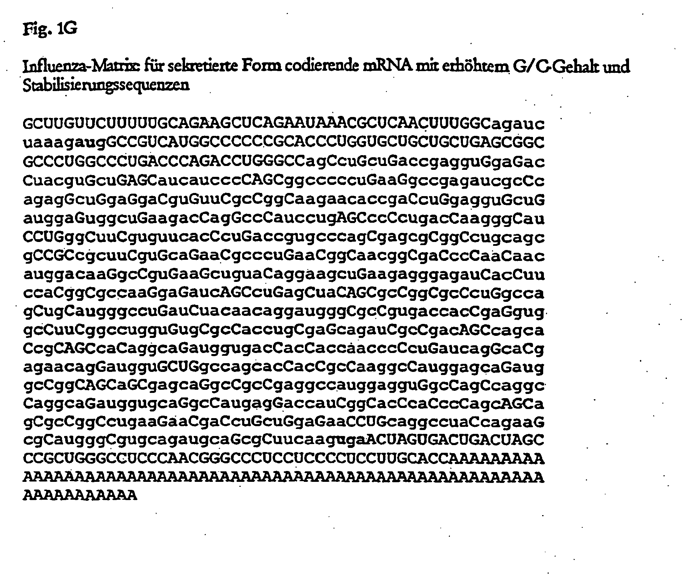

Fig. 1 zeigt Wildtyp- und modifizierte nicht-erfindungsgemäße Sequenzen für das Influenza-Matrix-Protein.
   Fig. 1A zeigt das Wildtyp-Gen und Fig. 1B zeigt die davon abgeleitete Aminosäuresequenz (Ein-Buchstaben-Code). Fig. 1C zeigt eine für das Influenza-Matrix-Protein codierende Gen-Sequenz, deren G/C-Gehalt gegenüber der Wildtyp-Sequenz erhöht ist. Fig. 1D zeigt die Sequenz eines Gens, das für eine sekretierte Form des Influenza-Matrix-Proteins codiert (einschließlich einer N-terminalen Signalsequenz), wobei der G/C-Gehalt der Sequenz gegenüber der Wildtyp-Sequenz erhöht ist. Fig. 1E zeigt eine für das Influenza-Matrix-Protein codierende mRNA, die im Vergleich zur Wildtyp-mRNA stabilisierende Sequenzen enthält. Fig. 1F zeigt eine für das Influenza-Matrix-Protein codierende nicht-erfindungsgemäße mRNA, die neben stabilisierenden Sequenzen einen erhöhten G/C-Gehalt aufweist. Fig. 1G zeigt ebenfalls eine modifizierte nicht-erfindungsgemäße mRNA, die für die sekretierte Form des Influenza-Matrix-Proteins codiert und im Vergleich zum Wildtyp stabilisierende Sequenzen und einen erhöhten GC-Gehalt aufweist. In Fig. 1A und Fig. 1C bis 1G sind die Start- und Stop-Codons fett hervorgehoben. Gegenüber der Wildtyp-Sequenz von Fig. 1A veränderte Nucleotide sind in den Figuren 1C bis 1G in Großbuchstaben dargestellt.

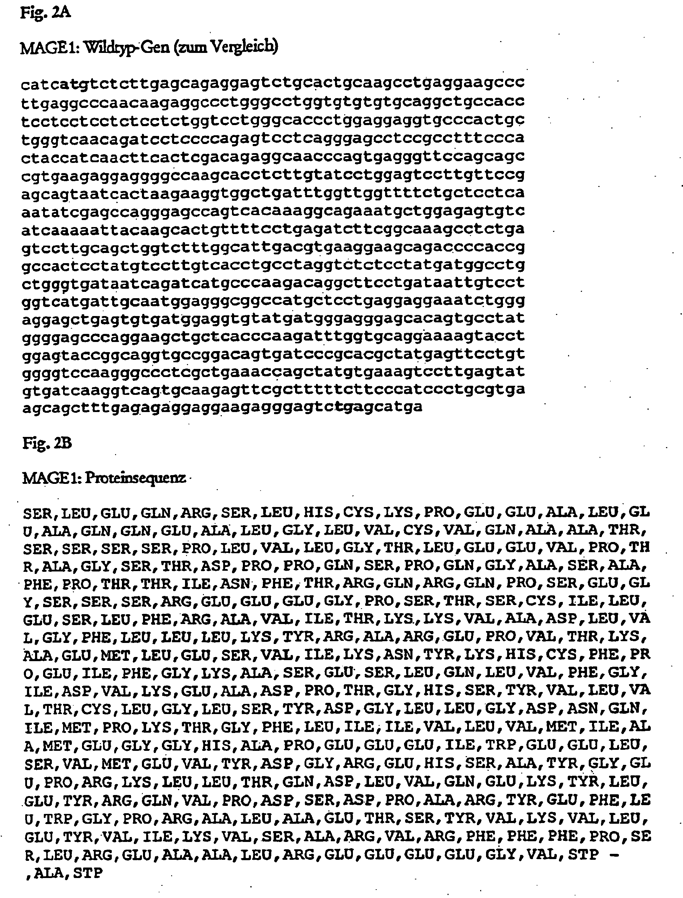

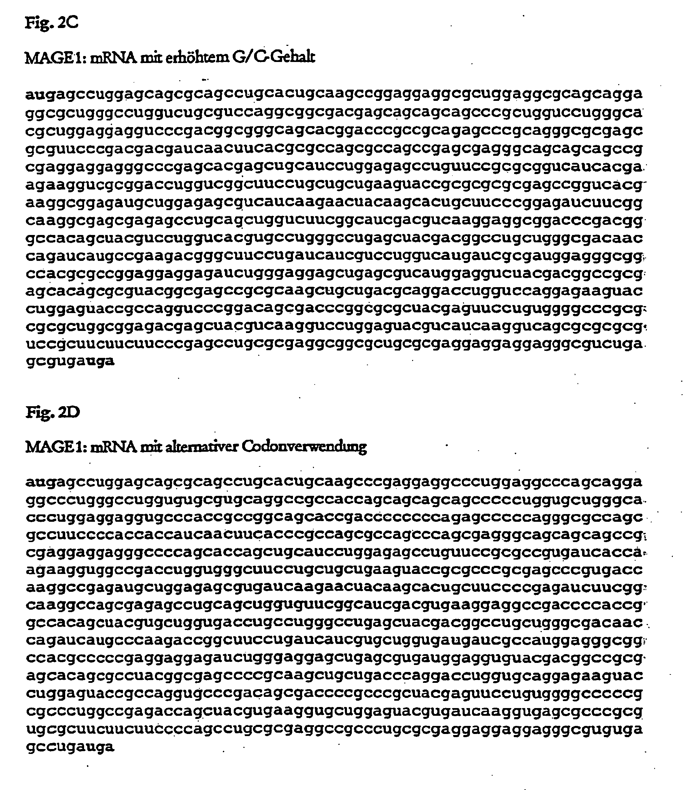

Fig. 2 zeigt Wildtyp- und modifizierte nicht-erfindungsgemäße Sequenzen, die für das Tumorantigen MAGE1 codieren.
   Fig. 2A zeigt die Sequenz des Wildtyp-Gens und Fig. 2B die davon abgeleitete Aminosäuresequenz (Drei-Buchstaben-Code) Fig. 2C zeigt eine für MAGE1 codierende modifizierte nicht-erfindungsgemäße mRNA, deren G/C-Gehalt gegenüber dem Wildtyp erhöht ist. Fig. 2D zeigt die Sequenz einer für MAGE1 codierenden modifizierten nicht-erfindungsgemäßen mRNA, bei der die Codon-Verwendung bezüglich der Codierung möglichst häufig in de Zelle vorkommender tRNA optimiert wurde. Start- und Stop-Codons sind jeweils fett gekennzeichnet.

Die folgenden Beispiele erläutern die vorliegende Erfindung näher, ohne sie einzuschränken.

### Beispiel 1

Als beispielhafte Ausführungsform des Verfahrens zu Ermittlung der Sequenz einer erfindungsgemäß modifizierten mRNA wurde ein Computerprogramm erstellt, das mit Hilfe des genetischen Codes bzw. dessen degenerativer Natur die Nucleotid-Sequenz einer beliebigen mRNA derart modifiziert, daß sich ein maximaler G/C-Gehalt in Verbindung mit der Verwendung von Codons, die für möglichst häufig in der Zelle vorkommende tRNAs codieren, ergibt, wobei die durch die modifizierte mRNA codierte Aminosäure-Sequenz gegenüber der nicht-modifizierten Sequenz identisch ist. Alternativ kann auch nur der G/C-Gehalt oder nur die Codonverwendung gegenüber der ursprünglichen Sequenz modifiziert werden.

Der Quellcode in Visual Basic 6.0 (eingesetzte Entwicklungsumgebung: Microsoft Visual Studio Enterprise 6.0 mit Servicepack 3) ist im Anhang angegeben.

### Beispiel 2

Mit Hilfe des Computerprogramms von Beispiel 1 wurde ein RNA-Konstrukt mit einer hinsichtlich Stabilisierung und Translationseffizienz optimierten Sequenz des lac-Z-Gens aus *E.coli* erstellt. Es konnte so ein G/C-Gehalt von 69% (im Vergleich zur Wildtyp-Sequenz von 51%; vgl. Kalnins et al., EMBO J. 1983, 2(4): 593-597) erzielt werden. Durch die Synthese überlappender Oligonucleotide, welche die modifizierte Sequenz aufweisen, wurde die optimierte Sequenz gemäß im Stand der Technik bekannter Verfahren hergestellt. Die endständigen Oligonucleotide wiesen die folgenden Restriktionsschnittstellen auf: Am 5'-Ende eine *Eco*RV- sowie am 3'-Ende eine *Bgl*II-Schnittstelle. Über Verdau mit *Eco*RV/*Bgl*II wurde die modifizierte lacZ-Sequenz in das Plasmid pT7Ts (GenBank-Zugriffsnummer U 26404; vgl. Lai et al., s.o.). pT7Ts enthält als nichttranslatierte Regionen Sequenzen aus dem β-Globingen von *Xenopus levis* jeweils an 5' und 3'. Das Plasmid wurde vor dem Einfügen der modifizierten lacZ-Sequenz mit den genannten Restriktionsenzymen geschnitten.

Das pT7Ts-lac-Z-Konstrukt wurde in Bakterien vermehrt und durch Phenol-Chloroform-Extraktion gereinigt. 2 µg des Konstrukts wurden *in vitro* mittels dem einem Fachmann bekannten Verfahren transkribiert, wodurch die modifizierte mRNA hergestellt wurde.

### Beispiel 3

Mit Hilfe des Computerprogramms von Beispiel 1 wurde das Gen für das Influenza-Matrix-Protein (Wildtyp-Sequenz vgl. Fig. 1A, abgeleitete Aminosäuresequenz Fig. 1B) optimiert. Dadurch wurde die in Fig. 1C dargestellte G/C-reiche Sequenzvariante erstellt. Ebenfalls wurde eine für die sekretierte Form des Influenza-Matrix-Proteins codierende G/C-reiche Sequenz, die für eine N-terminale Signalsequenz codiert, ermittelt (vgl. Fig. 1D). Die sekretierte Form des Influenza-Matrix-Proteins hat den Vorteil, daß sie eine im Vergleich zur nicht-sekretierten Form erhöhte Immunogenizität aufweist.

Ausgehend von den optimierten Sequenzen wurden entsprechende nicht-erfindungsgemäße mRNA-Moleküle entworfen. Die hinsichtlich G/C-Gehalt und Codonverwendung optimierte mRNA für das Influenza-Matrix-Protein wurde zusätzlich mit stabilisierenden Sequenzen im 5'- und 3'-Bereich (die Stabilisierungssequenzen stammen aus den 5'- bzw. 3'-UTRs des β-Globin-mRNA von *Xenupus laevis*; vgl. pT7Ts-Vektor in Lai et al., s.o.) ausgestattet (vgl. Fig. 1E und 1F). Desgleichen wurde auch die für die sekretierte Form des Influenza-Matrix-Proteins codierende mRNA im translatierten Bereich sequenzoptimiert und mit den genannten stabilisierenden Sequenzen versehen (vgl. Fig. 1G).

### Beispiel 4

Mit Hilfe des Computerprogramms von Beispiel 1 wurde die für das Tumorantigen MA-GE1 codierende mRNA modifiziert. Dadurch wurde die in Fig. 2C gezeigte Sequenz ermittelt, die im Vergleich zur Wildtyp-Sequenz (275 G, 244 G) einen um 24% höheren G/C-Gehalt aufweist (351 G, 291 C). Des weiteren wurde die Wildtyp-Sequenz durch alternative Codonverwendung hinsichtlich der Translationseffizienz aufgrund der Codierung von in der Zelle häufiger vorkommenden tRNAs verbessert (vgl. Fig. 2D). Durch die alternative Codonverwendung wurde der G/C-Gehalt ebenfalls um 24% erhöht.

### Anhang: Quelltext eines erfindungsgemäßen Computerprogramms

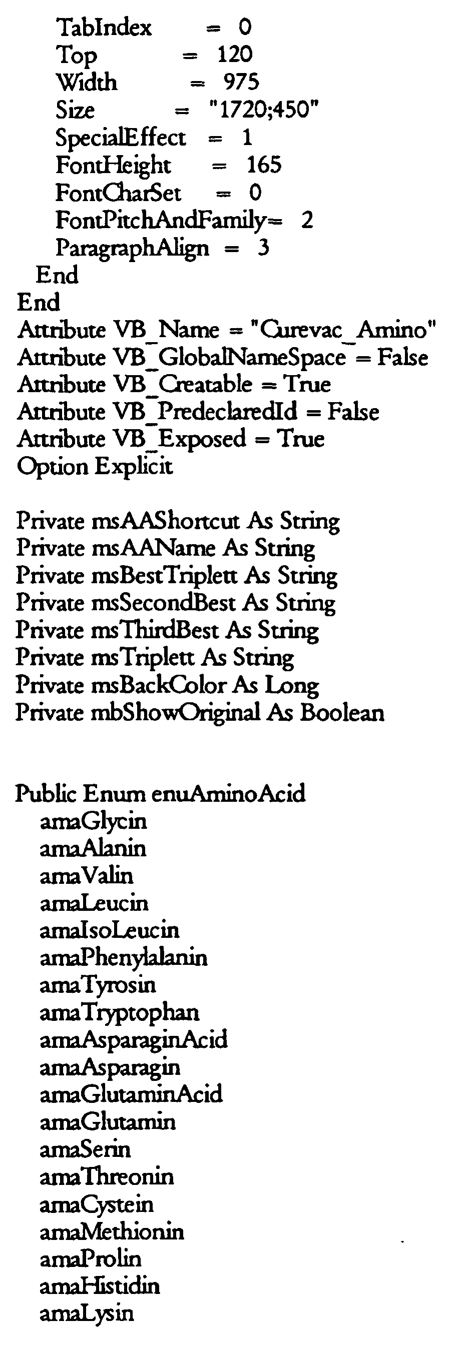

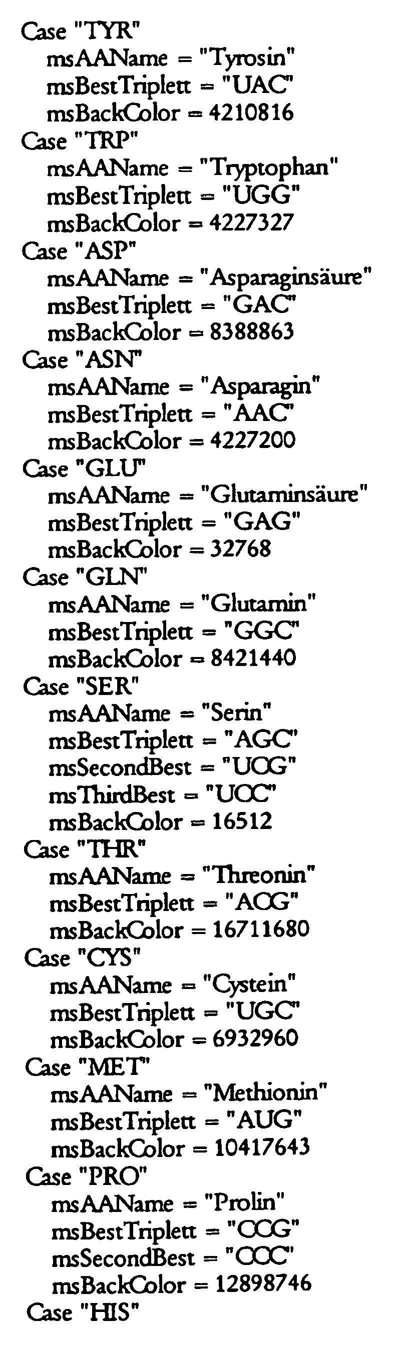

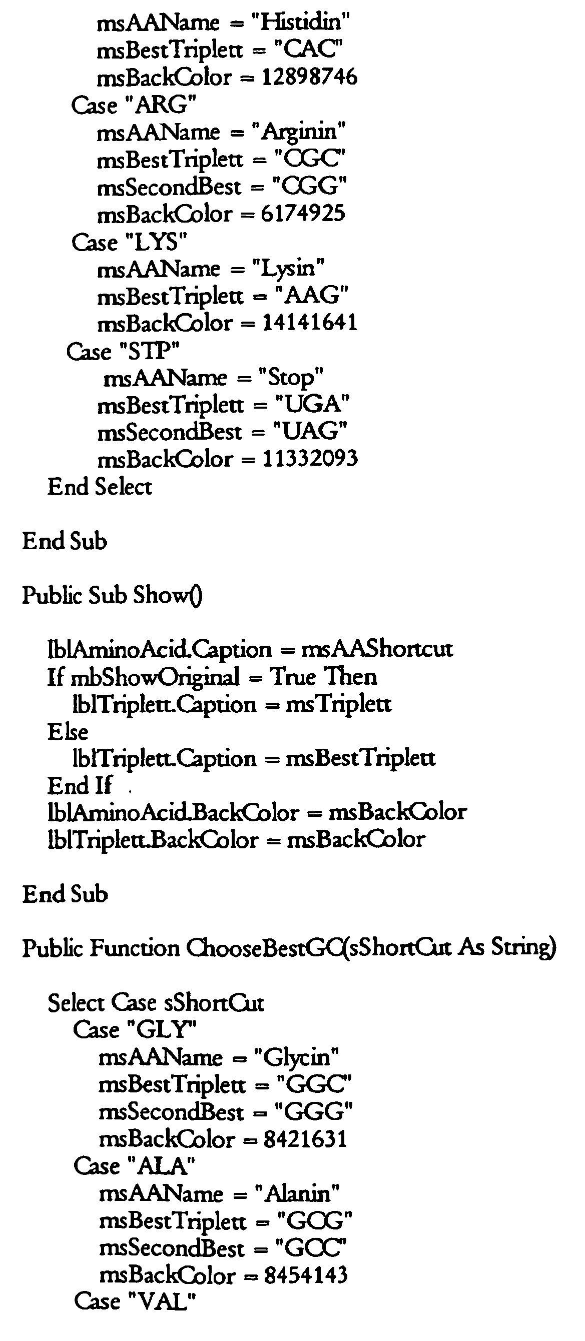

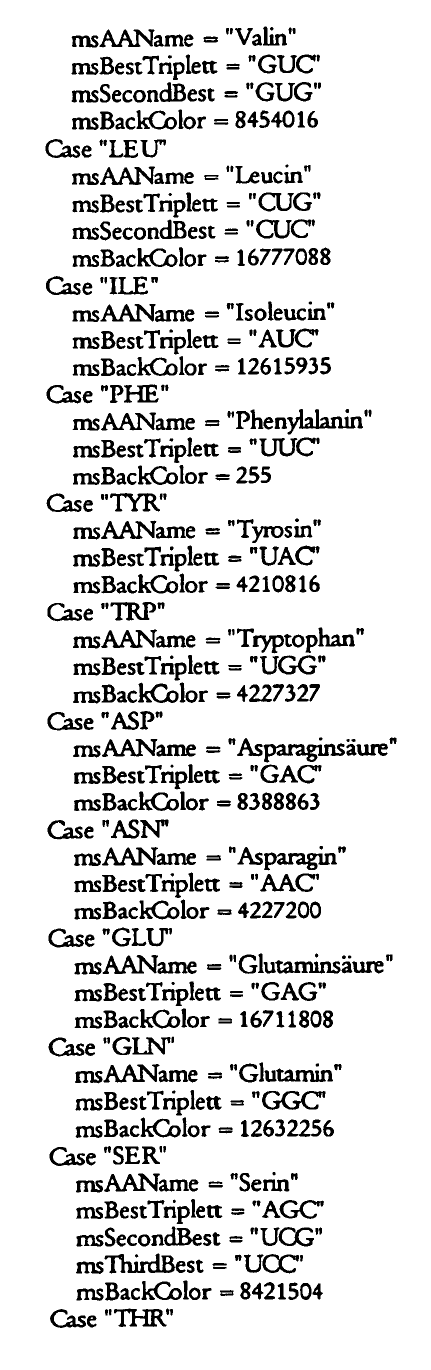

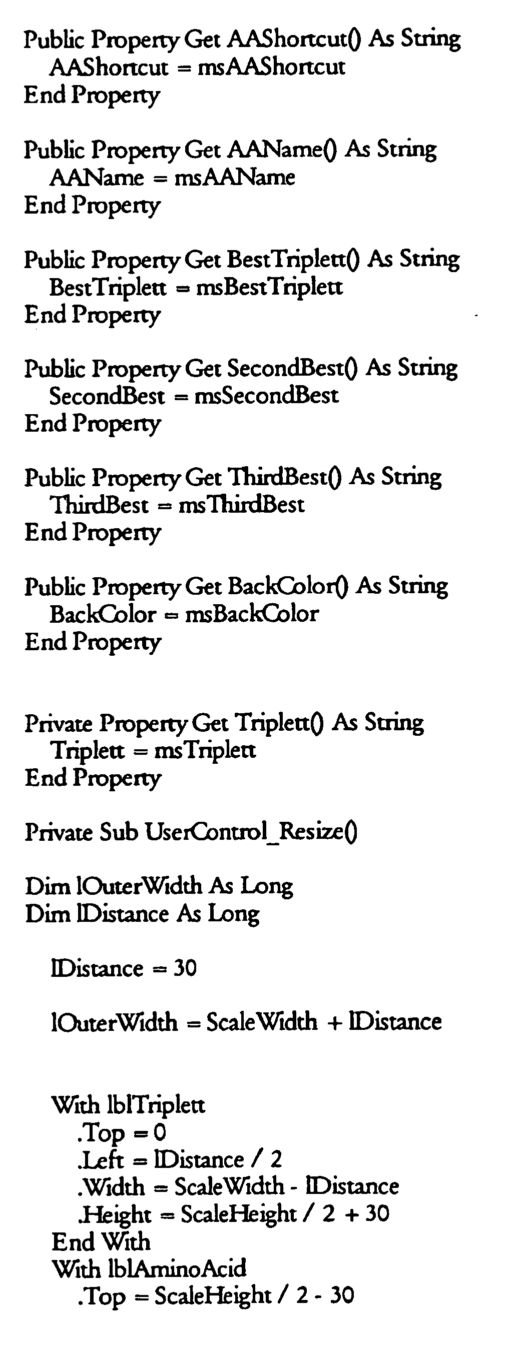

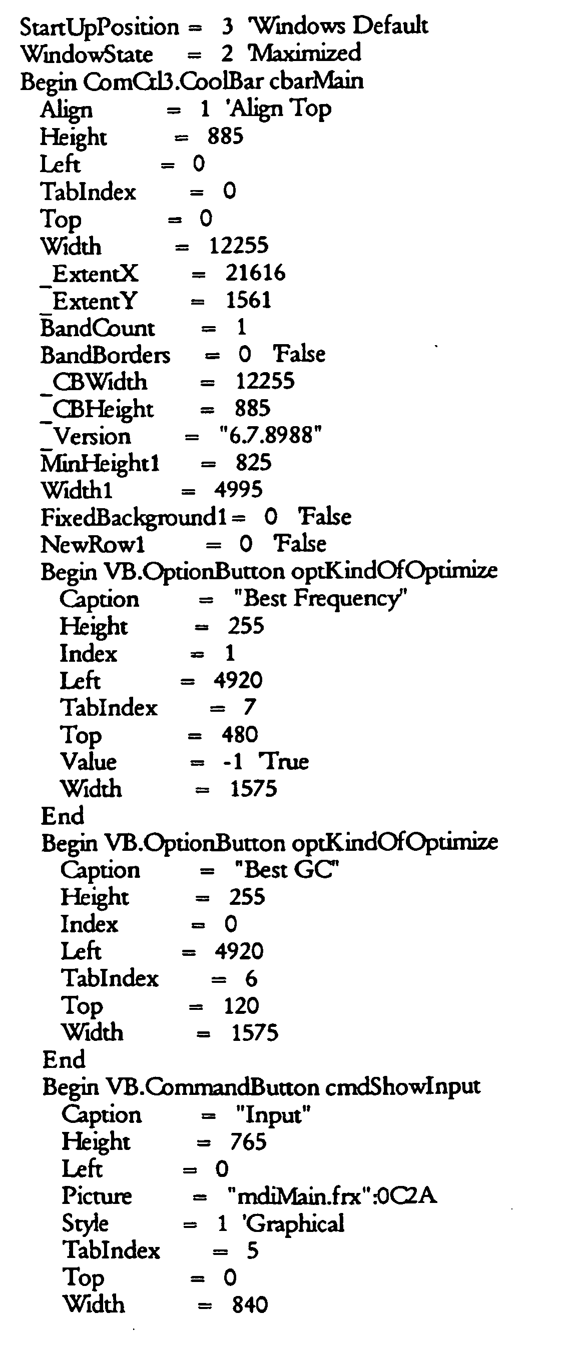

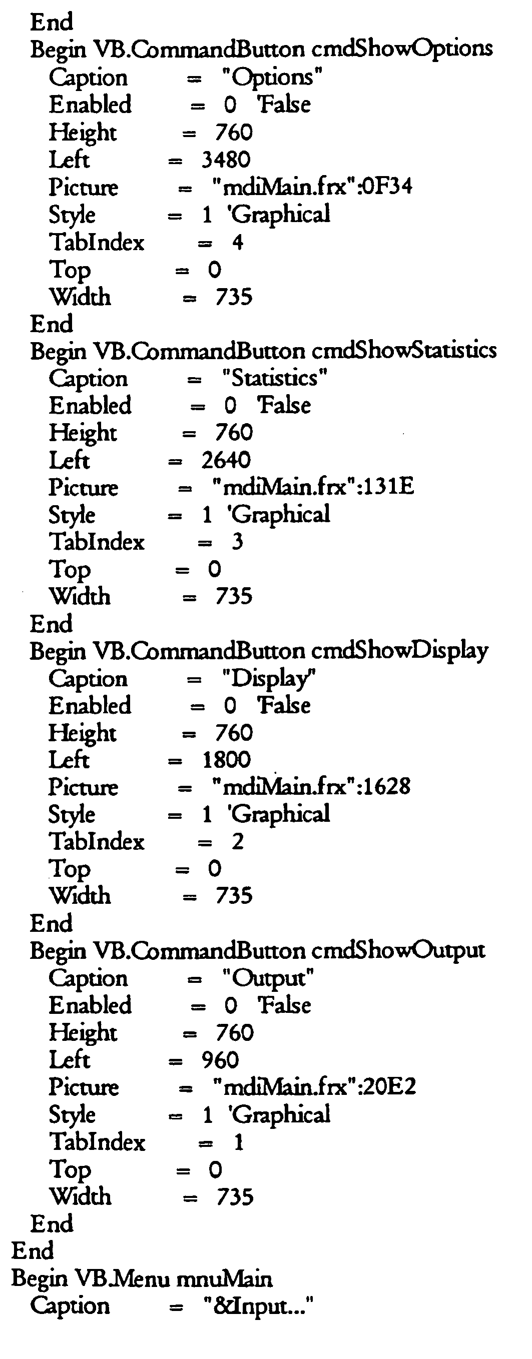

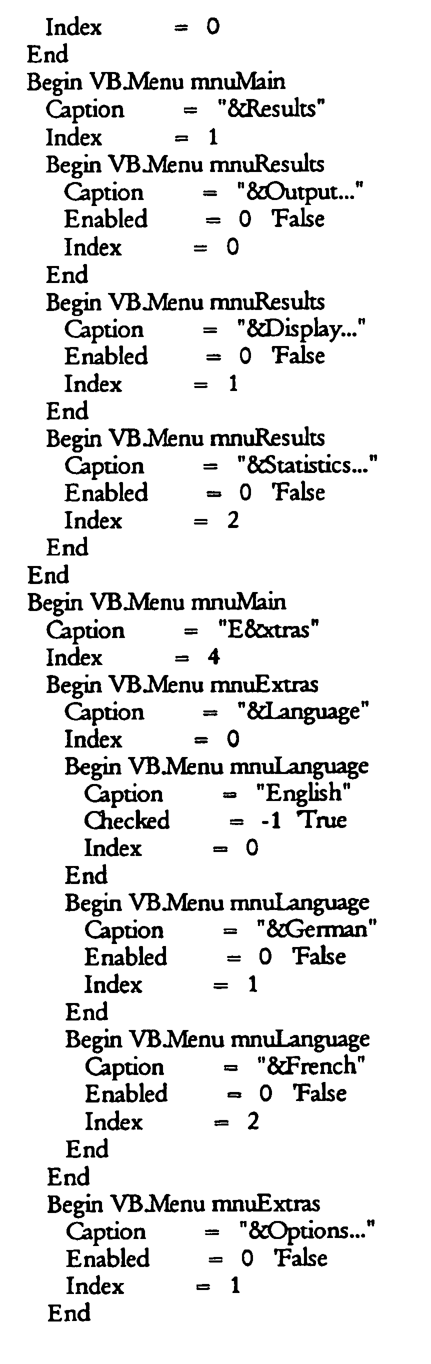

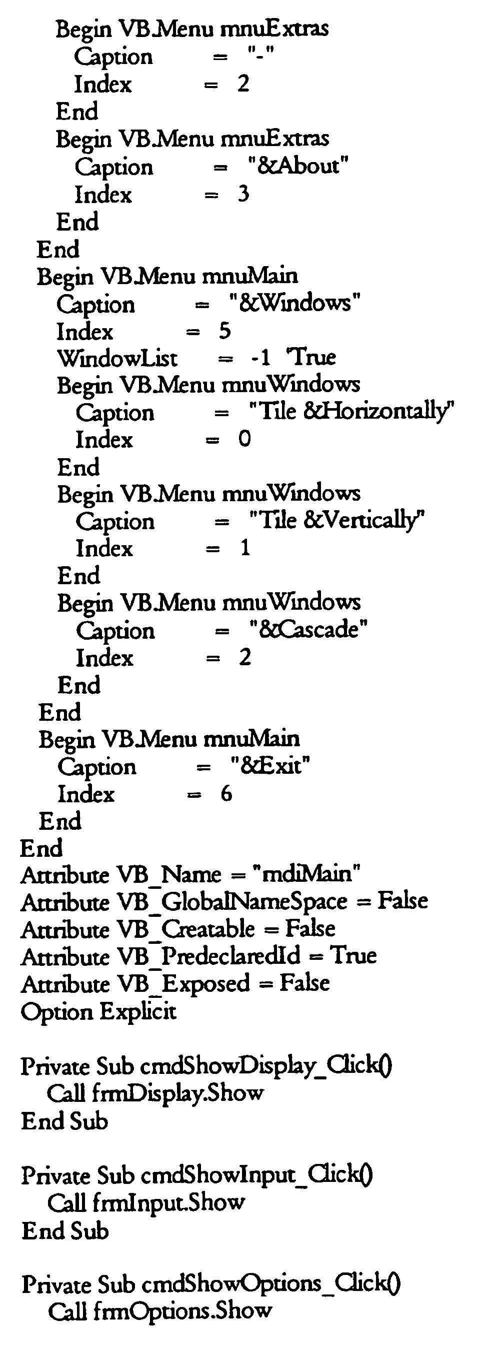

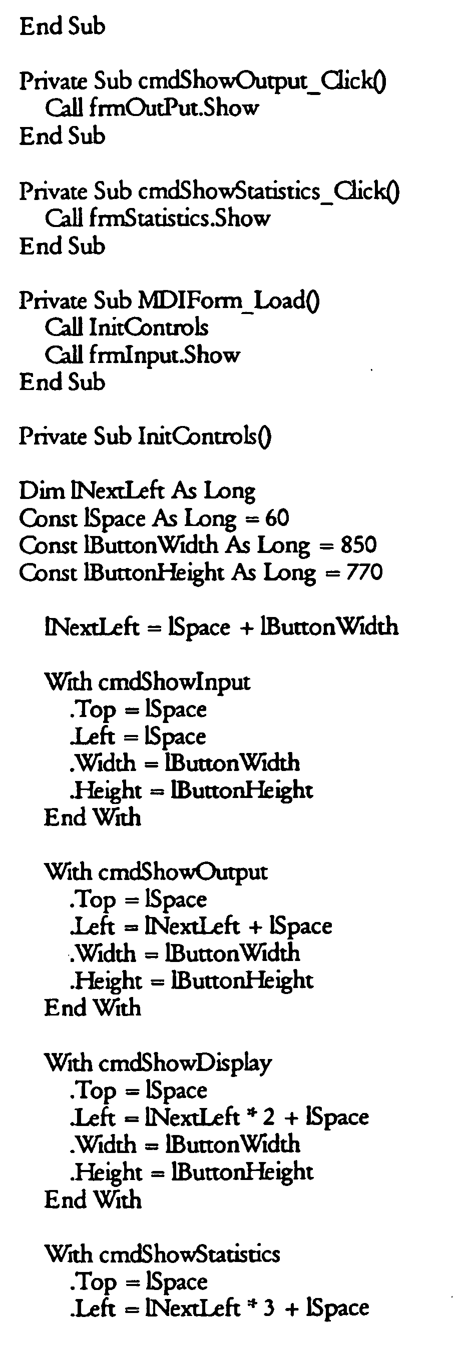

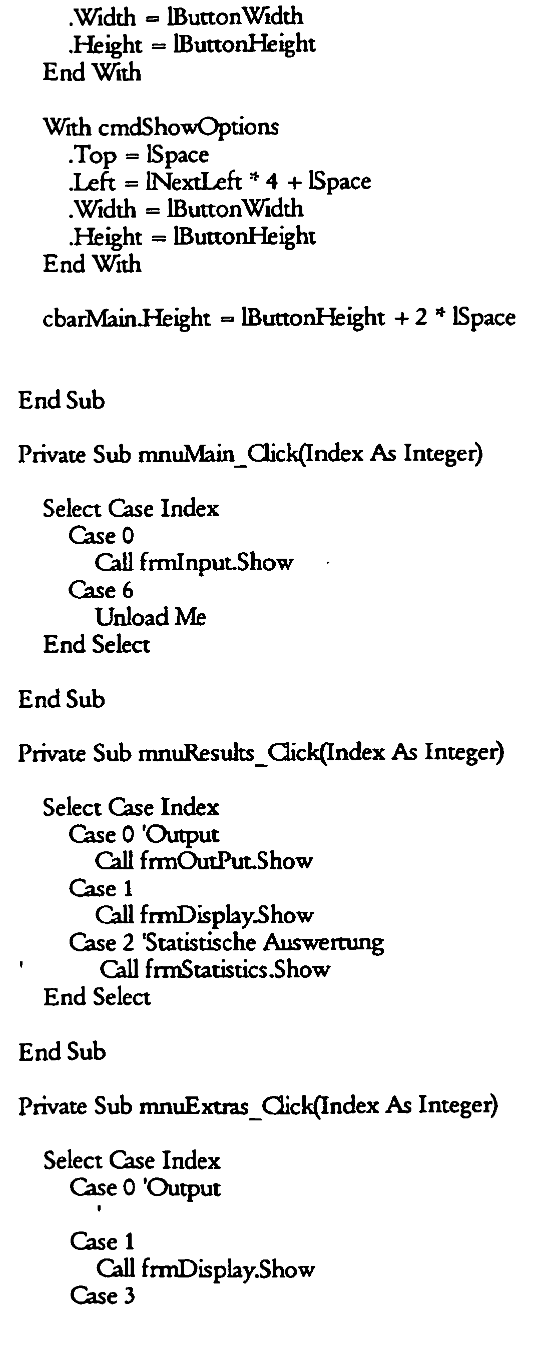

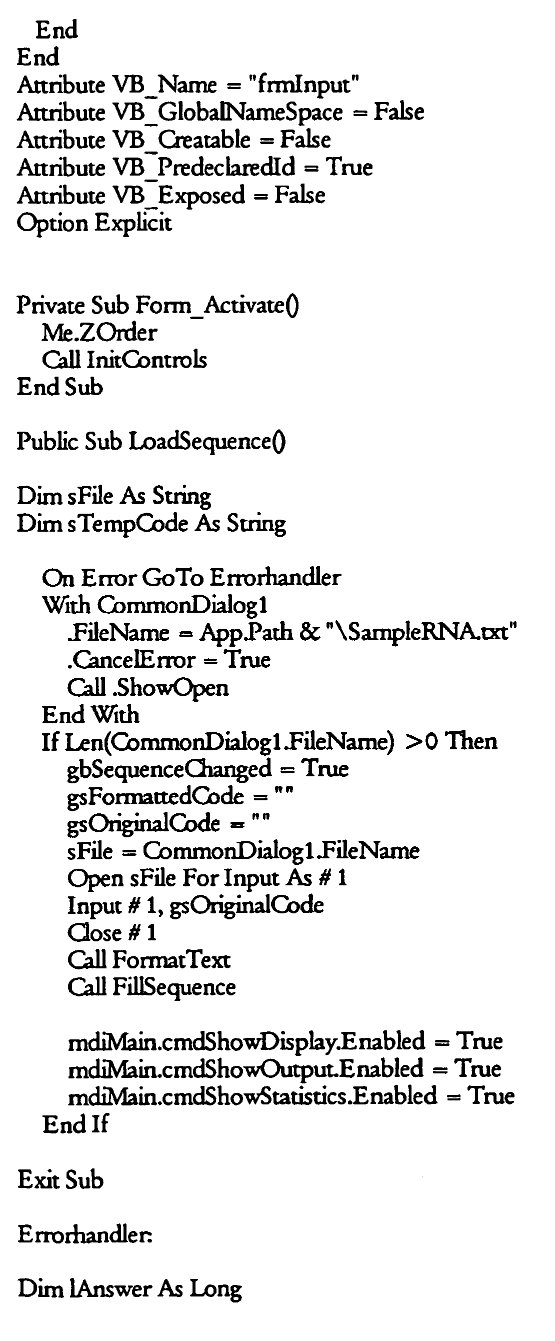

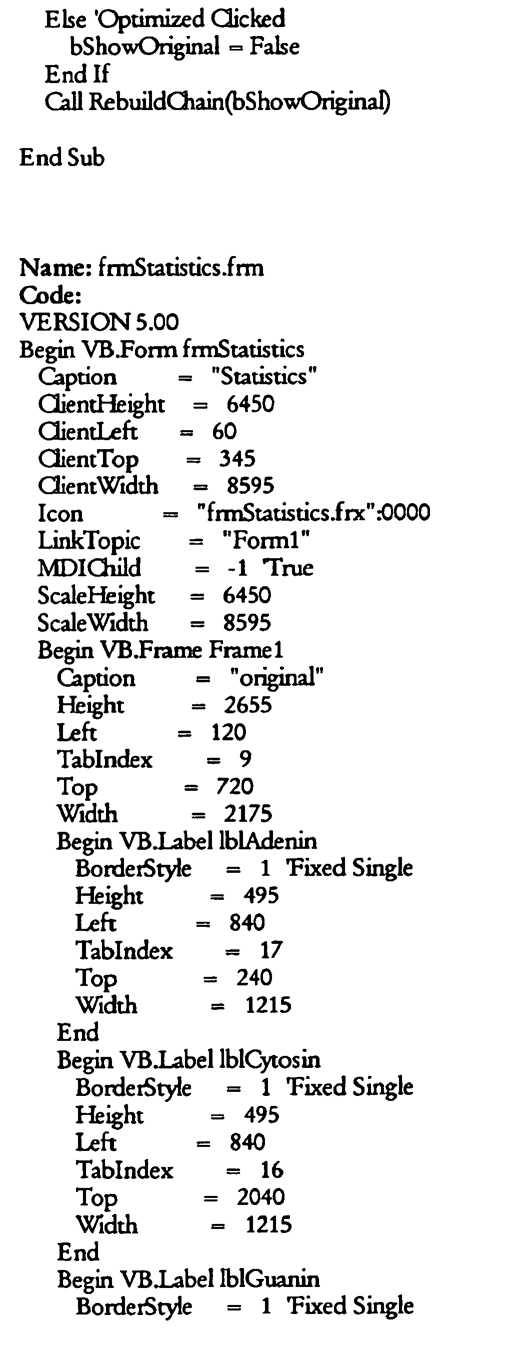

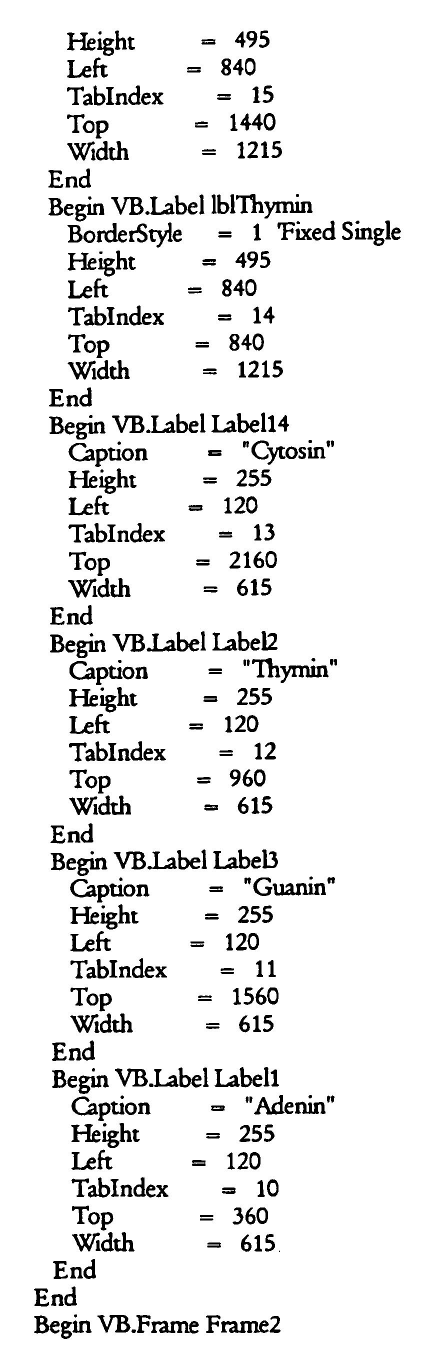

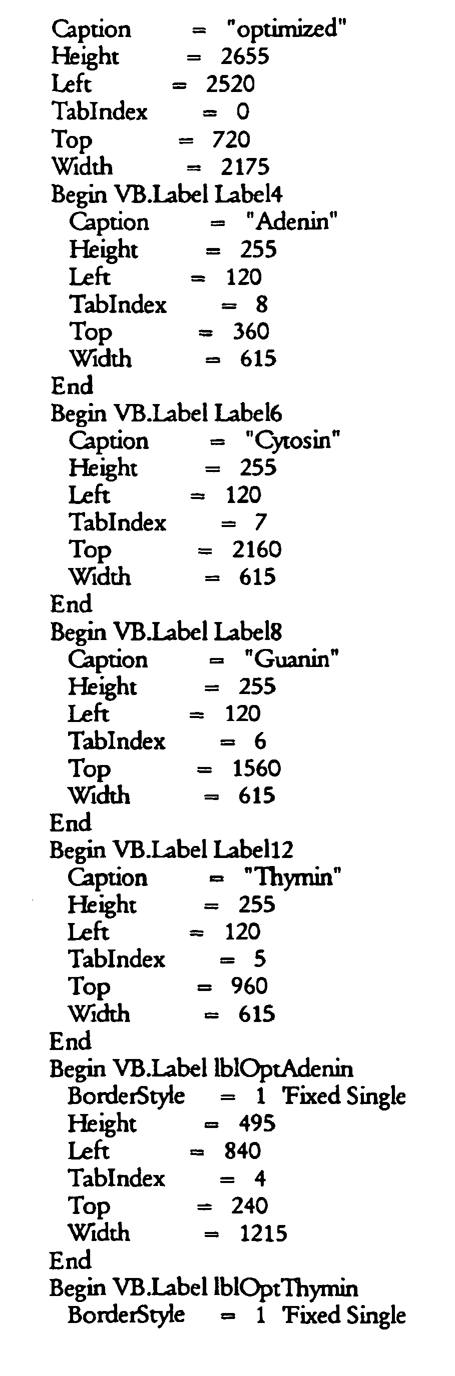

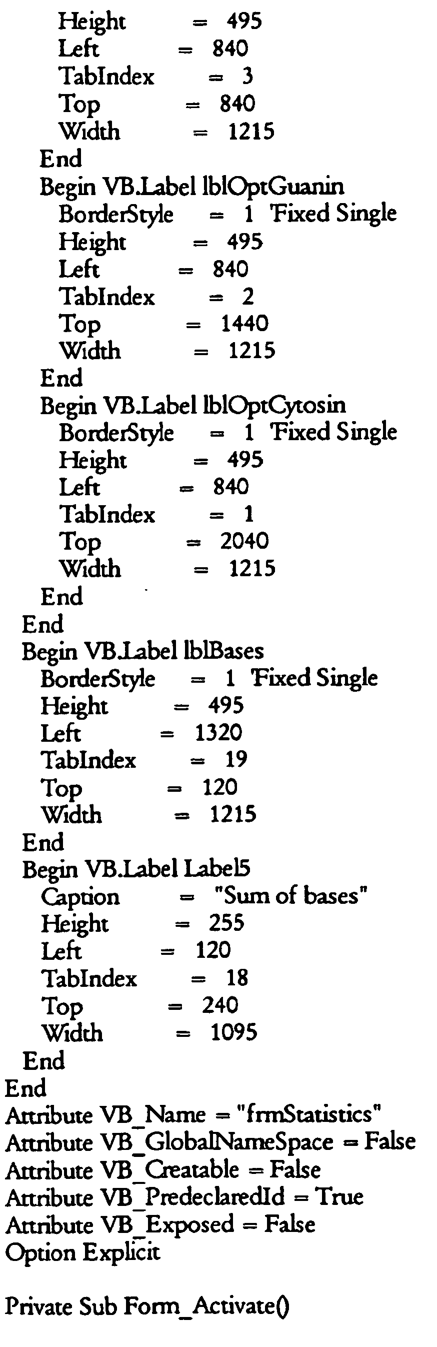

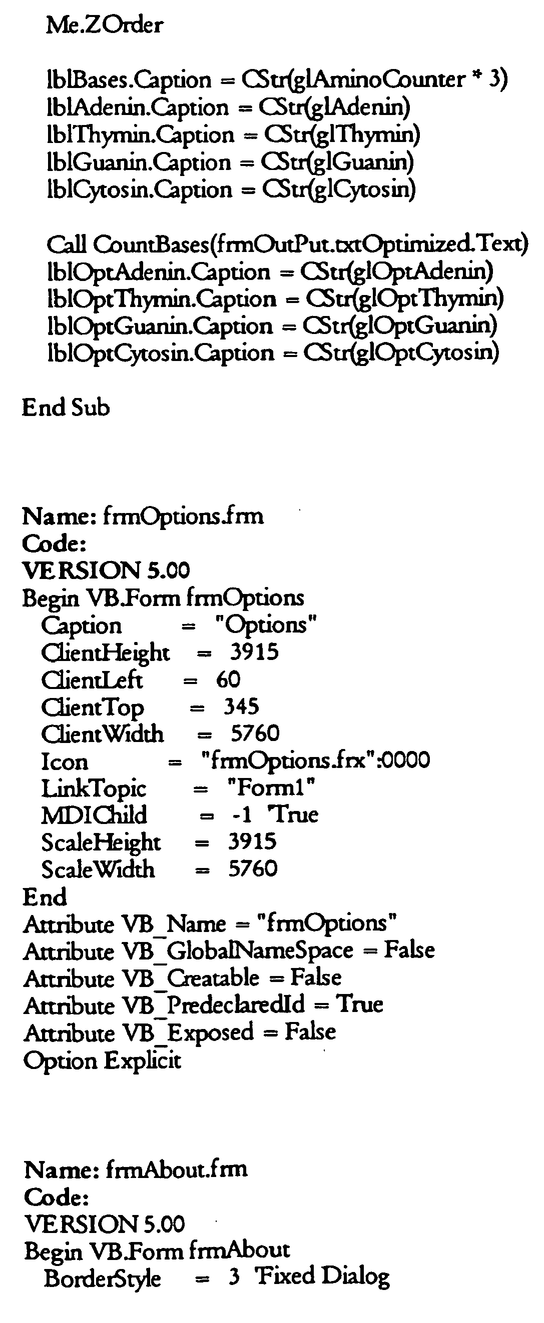

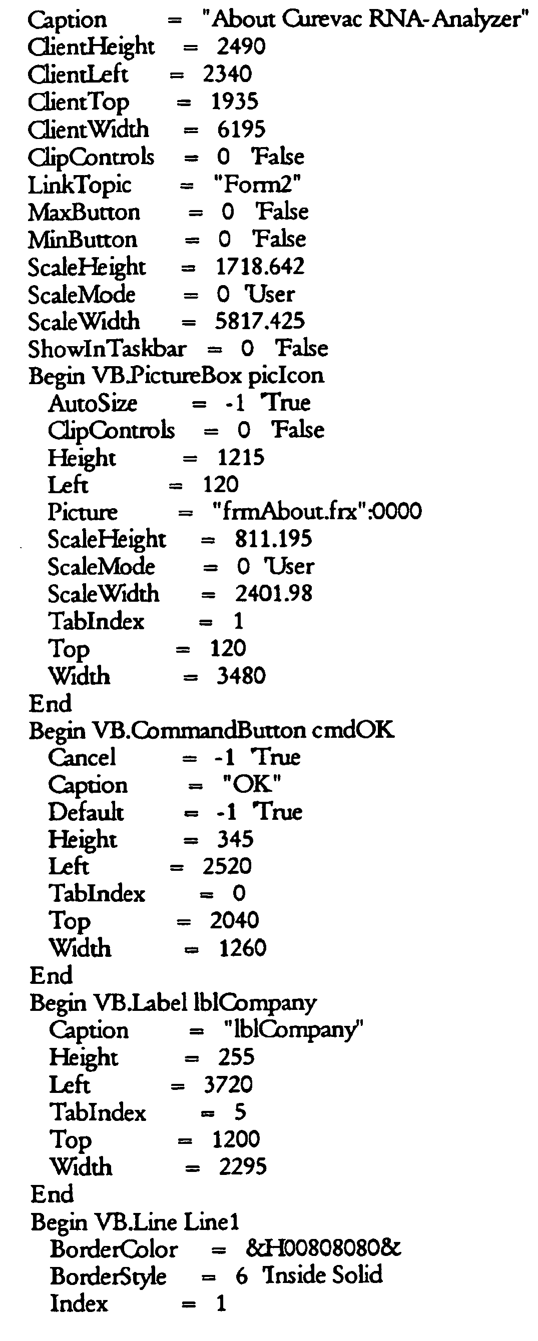

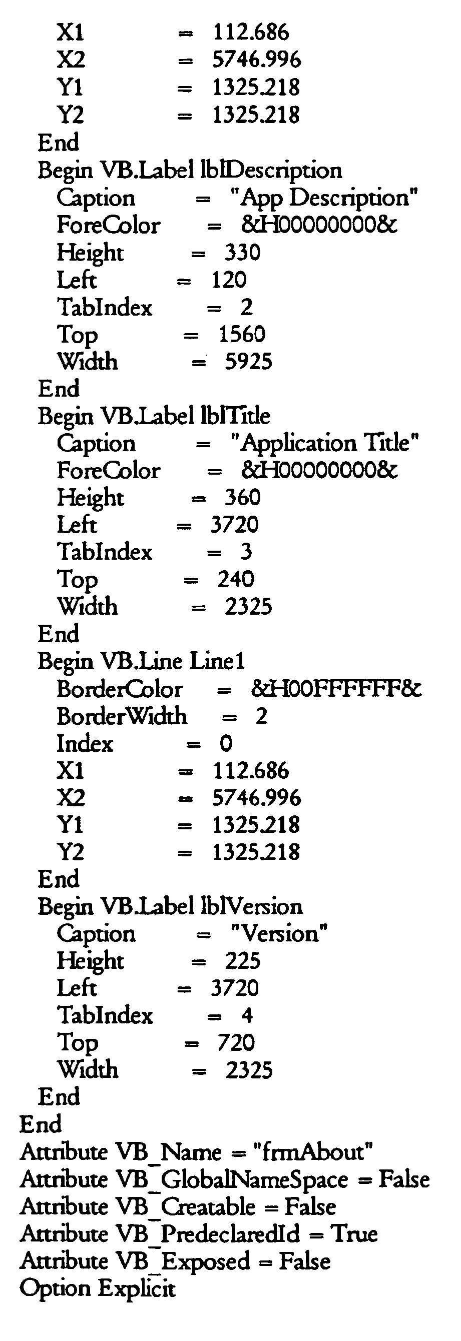

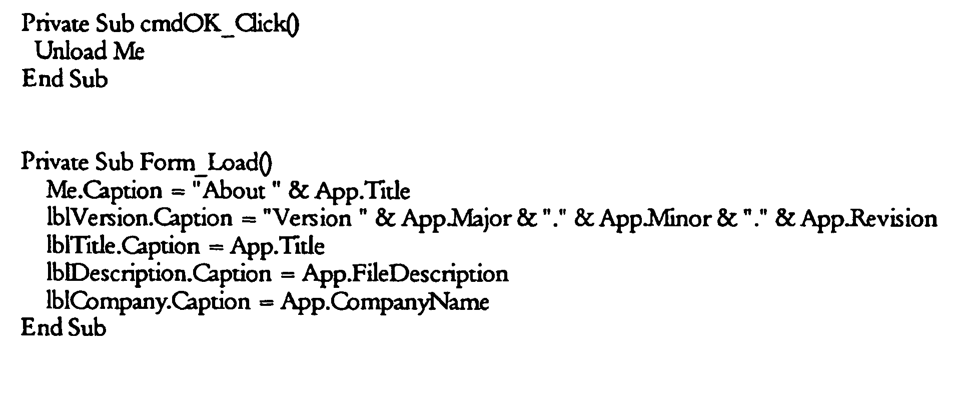

## Patentansprüche

1. Verwendung einer modifizierten mRNA, die für mindestens ein biologisch wirksames Peptid oder Polypeptid codiert, **dadurch gekennzeichnet, dass** der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA ist und die codierte Aminosäuresequenz gegenüber dem Wildtyp unverändert ist, zur Herstellung eines Medikaments zur Gentherapie.

2. Verwendung einer modifizierten mRNA nach Anspruch 1, **dadurch gekennzeichnet, dass** der G/C-Gehalt des für das Peptid oder Polypeptid codierenden Bereichs der modifizierten mRNA um mindestens 7%-Punkte, bevorzugt mindestens 15%-Punkte, größer als der G/C-Gehalt des codierenden Bereichs der für das Peptid oder Polypeptid codierenden Wildtyp-mRNA ist.

3. Verwendung einer modifizierten mRNA nach Anspruch 1 oder 2, **dadurch gekennzeichnet, dass** der für das Peptid oder Polypeptid codierende Bereich der modifizierten mRNA derart verändert ist, dass sich ein maximaler G/C-Gehalt in Verbindung mit den Codons ergibt, die für relative häufige tRNAs codieren.

4. Verwendung einer modifizierten mRNA nach einem der Ansprüche 1 bis 3, **dadurch gekennzeichnet, dass** die modifizierte mRNA eine 5'-Cap-Struktur und/oder einen polyA-Schwanz von mindestens 70 Nucleotiden und/oder eine IRES und/oder eine 5'-Stabilisierungssequenz und/oder eine 3'-Stabilisierungssequenz aufweist.

5. Verwendung einer modifizierten mRNA nach einem der Ansprüche 1 bis 4, **dadurch gekennzeichnet, dass** die modifizierte mRNA mindestens ein Analoges natürlich vorkommender Nucleotide aufweist.

6. Verwendung einer modifizierten mRNA nach Anspruch 5, **dadurch gekennzeichnet, dass** das Analoge aus der Gruppe, bestehend aus Phosphorthioaten, Phosphoramidaten, Peptidnucleotiden, Methylphosphonaten, 7-Deazaguanosin, 5-Methylcytosin und Inosin, ausgewählt ist.

7. Verwendung einer modifizierten mRNA nach einem der Ansprüche 1 bis 6, **dadurch gekennzeichnet, dass** die mRNA für ein Polypeptid codiert, welches in dem zu behandelnden Patienten nicht oder nur unzureichend oder fehlerhaft gebildet wird.

8. Verwendung einer modifizierten mRNA nach einem der Ansprüche 1 bis 7, **dadurch gekennzeichnet, dass** die modifizierte mRNA für Dystrophin, Enzyme, die bei metabolischen Erkrankungen fehlen oder fehlerhaft sind, oder Enzyme, die an der Synthese von Neurotransmittern beteiligt sind, codiert.

9. Verwendung einer modifizierten mRNA nach einem der Ansprüche 1 bis 7, **dadurch gekennzeichnet, dass** die mRNA ein Peptid oder Protein codiert, das an Zelloberflächen-Rezeptoren bindet.

10. Verwendung einer modifizierten mRNA nach einem der Ansprüche 1 bis 7 oder 9, **dadurch gekennzeichnet, dass** die mRNA für Wachstumsfaktoren oder Wachstumshormone codiert.

11. Verwendung einer modifizierten mRNA nach Anspruch 7 oder 9, **dadurch gekennzeichnet, dass** die mRNA für den Gewebeplasminogenaktivator, Insulin, Interferone, GM-CSF, Erythropoietin, Tyrosinhydroxylase, DOPA-Decarboxylase oder α-1-Antitrypsin codiert.

12. Verwendung einer modifizierten mRNA nach einem der Ansprüche 1 bis 7, **dadurch gekennzeichnet, dass** die mRNA mit einem kationischen Peptid oder Protein assoziiert oder daran gebunden ist, insbesondere Protamin, Poly-L-Lysin oder Historie.

13. Verwendung einer modifizierten mRNA nach einem der Ansprüche 1 bis 9, **dadurch gekennzeichnet, dass** die mRNA zusätzlich für mindestens ein Cytokin codiert.

14. Verwendung einer modifizierten mRNA nach einem der Ansprüche 1 bis 13, in Verbindung mit einem pharmazeutisch verträglichen Träger und/oder Vehikel.

15. Verwendung einer modifizierten mRNA nach einem der Ansprüche 1 bis 14, zur Geweberegeneration oder zur Behandlung von degenerativen, insbesondere neurodegenerativen Erkrankungen.

16. Verwendung einer modifizierten mRNA nach einem der Ansprüche 1 bis 14, zur Behandlung von Morbus Alzheimer, Morbus Parkinson oder Arthrose.

17. Verwendung einer modifizierten mRNA nach Anspruch 15 oder 16, **dadurch gekennzeichnet dass** die mRNA für Wachstumsfaktoren aus der TGF-β-Familie codiert.

18. Verwendung einer modifizierten mRNA nach Anspruch 15 oder 16, **dadurch gekennzeichnet, dass** die mRNA für EGF, FGF, PDGF, BMP, GDNF, BDNF, GDF und neurotrophe Faktoren, bspw. NGF oder Neutrophine, codiert.

19. Modifizierte mRNA, die für mindestens ein biologisch wirksames Peptid oder Polypeptid codiert, welches in dem zu behandelnden Patienten nicht oder nur unzureichend oder fehlerhaft gebildet wird, **dadurch gekennzeichnet, dass** der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA ist und die codierte Aminosäuresequenz gegenüber dem Wildtyp unverändert ist.

20. Modifizierte mRNA nach Anspruch 19, wobei die modifizierte mRNA Enzyme, die bei metabolischen Erkrankungen fehlen bzw. fehlerhaft sind, oder für Enzyme, die an der Synthese von Neurotransmittern beteiligt sind, codiert.

21. Modifizierte mRNA nach Anspruch 19, wobei die modifizierte mRNA für Zelloberflächen-Rezeptoren codiert.

22. Modifizierte mRNA nach Anspruch 19, **dadurch gekennzeichnet, dass** die modifizierte mRNA für Proteine, die an Zelloberflächen-Rezeptoren binden oder extrazellulär wirken, ausgewählt werden aus der Gruppe bestehend aus Gewebeplasminogenaktivator, Wachstumshormone, Insulin, Interferone, Granulozyten-Makrophagen-Koloniestimulierungsfaktor, Wachstumsfaktoren und Erythropoietin, codiert.

23. Pharmazeutische Zusammensetzung, enthaltend eine modifizierte mRNA nach einem der Ansprüche 19 bis 22 in Verbindung mit einem pharmazeutisch verträglichen Träger und/oder Vehikel.

## Claims

1. Use of a modified mRNA coding for at least one biologically active peptide or polypeptide, **characterised in that** the G/C content of the region of the modified mRNA coding for the peptide or polypeptide is increased compared to the G/C content of the coding region of the wild type mRNA coding for the peptide or polypeptide, and the encoded amino acid sequence is unchanged as compared to the wild type, for the preparation of a medicament for gene therapy.

2. Use of a modified mRNA according to claim 1, **characterised in that** the G/C content of the region of the modified mRNA coding for the peptide or polypeptide is increased by at least 7 % points, preferably at least 15 % points, compared to the G/C content of the coding region of the wild type mRNA coding for the peptide or polypeptide.

3. Use of a modified mRNA according to claim 1 or 2, **characterised in that** the region of the modified mRNA coding for the peptide or polypeptide is altered in such a way as to produce a maximum G/C content in conjunction with the codons that code for the relatively frequent tRNAs.

4. Use of a modified mRNA according to one of claims 1 to 3, **characterised in that** the modified mRNA comprises a 5' cap structure and/or a poly-A tail of at least 70 nucleotides and/or an IRES and/or a 5' stabilisation sequence and/or a 3' stabilisation sequence.

5. Use of a modified mRNA according to one of claims 1 to 4, **characterised in that** the modified mRNA comprises at least one analogue of naturally occurring nucleotides.

6. Use of a modified mRNA according to claim 5, **characterised in that** the analogue is selected from the group consisting of phosphorus thioates, phosphorus amidates, peptide nucleotides, methylphosphonates, 7-deazaguanosine, 5-methylcytosine and inosine.

7. Use of a modified mRNA according to one of claims 1 to 6, **characterised in that** the mRNA codes for a polypeptide which is not formed or is only insufficiently or defectively formed in the patient to be treated.

8. Use of a modified mRNA according to one of claims 1 to 7, **characterised in that** the modified mRNA codes for dystrophin, enzymes that are lacking or defective in metabolic diseases, or enzymes that are involved in the synthesis of neurotransmitters.

9. Use of a modified mRNA according to one of claims 1 to 7, **characterised in that** the mRNA codes for a peptide or protein that binds to cell surface receptors.

10. Use of a modified mRNA according to one of claims 1 to 7 or 9, **characterised in that** the mRNA codes for growth factors or growth hormones.

11. Use of a modified mRNA according to claim 7 or 9, **characterised in that** the mRNA codes for the tissue plasminogen activator, insulin, interferones, GM-CSF, erythropoietin, tyrosine hydroxylase, DOPA decarboxylase, or α-1 antitrypsin.

12. Use of a modified mRNA according to one of claims 1 to 7, **characterised in that** the mRNA is associated with a cationic peptide or protein or is bound thereto, in particular protamine, poly-L lysine, or histone.

13. Use of a modified mRNA according to one of claims 1 to 9, **characterised in that** the mRNA in addition codes for at least one cytokine.

14. Use of a modified mRNA according to one of claims 1 to 13 in combination with a pharmaceutically acceptable carrier and/or vehicle.

15. Use of a modified mRNA according to one of claims 1 to 14 for tissue regeneration or for the treatment of degenerative, in particular neurodegenerative diseases.

16. Use of a modified mRNA according to one of claims 1 to 14 for the treatment of Alzheimer's disease, Parkinson's disease or arthrosis.

17. Use of a modified mRNA according to claim 15 or 16, **characterised in that** the mRNA codes for growth factors of the TGF-β family.

18. Use of a modified mRNA according to claim 15 or 16, **characterised in that** the mRNA codes for EGF, FGF, PDGF, BMP, GDNF, BDNF, GDF and neuthrophic factors, such as NGF or neutrophines.

19. Modified mRNA coding for at least one biologically active peptide or polypeptide which is not formed or is only insufficiently or defectively formed in the patient to be treated, **characterised in that** the G/C content of the region of the modified mRNA coding for the peptide or polypeptide is increased compared to the G/C content of the coding region of the wild type mRNA coding for the peptide or polypeptide, and the encoded amino acid sequence is unchanged as compared to the wild type.

20. Modified mRNA according to claim 19, wherein the modified mRNA codes for enzymes that are lacking or are defective in metabolic diseases, or enzymes that are involved in the synthesis of neurotransmitters.

21. Modified mRNA according to claim 19, wherein the modified mRNA codes for cell surface receptors.

22. Modified mRNA according to claim 19, wherein the modified mRNA codes for proteins that bind to cell surface receptors or act in an extracellular manner, selected from the group consisting of tissue plasminogen activator, growth hormones, insulin, interferones, granulocyte macrophage colony stimulating factor, growth factors, and erythropoietin.

23. Pharmaceutical composition containing a modified mRNA according to one of claims 19 to 22 in combination with a pharmaceutically acceptable carrier and/or vehicle.

## Revendications

1. Utilisation d'un ARNm modifié qui code pour au moins un peptide ou polypeptide biologiquement actif, **caractérisé en ce que** la teneur en G/C de la région de l'ARNm modifié codant pour le peptide ou polypeptide est augmentée par rapport à la teneur en G/C de la région codante de l'ARNm de type sauvage codant pour le peptide ou polypeptide, et **en ce que** la séquence d'acide aminés codée est inchangée par rapport au type sauvage, pour la préparation d'un médicament destiné à la thérapie génique.

2. Utilisation d'un ARNm modifié selon la revendication 1, **caractérisé en ce que** la teneur en G/C de la région de l'ARNm modifié codant pour le peptide ou polypeptide est augmentée d'au moins 7 points, préféré d'au moins 15 points, en pourcentage par rapport à la teneur en G/C de la région codante de l'ARNm de type sauvage codant pour le peptide ou polypeptide.

3. Utilisation d'un ARNm modifié selon la revendication 1 ou 2, **caractérisé en ce que** la région de l'ARNm modifié codant pour le peptide ou polypeptide est modifiée de telle sorte qu'il s'ensuit une teneur en G/C maximale en relation avec les codons qui codent pour des ARNt relativement frequents.

4. Utilisation d'un ARNm modifié selon l'une des revendications 1 à 3, **caractérisé en ce que** l'ARNm modifié comporte une structure de coiffe 5' et/ou une queue poly-A d'au moins 70 nucleotides et/ou une séquence IRES et/ou une séquence de stabilisation 5' et/ou une séquence de stabilisation 3'.

5. Utilisation d'un ARNm modifié selon l'une des revendications 1 à 4, **caractérisé en ce que** l'ARNm modifié comporte au moins un analogue de nucléotides se rencontrant dans la nature.

6. Utilisation d'un ARNm modifié selon la revendication 5, **caractérisé en ce que** l'analogue est choisi dans le groupe consistant en des phosphothioates, des phosphoamidates, des nucléotides peptidiques, des phosphonates de méthyle, la 7-déazaguanosine, la 5-méthylcytosine et l'inosine.

7. Utilisation d'un ARNm modifié selon l'une des revendications 1 à 6, **caractérisé en ce que** l'ARNm code pour un polypeptide lequel n'est pas formé ou est formé seulement de façon insuffisante ou erronée, chez le patient à traiter.

8. Utilisation d'un ARNm modifié selon l'une des revendications 1 à 7, **caractérisé en ce que** l'ARNm modifié code pour la dystrophine, les enzymes qui manquent ou bien sont défectueuses dans les maladies métaboliques, ou les enzymes qui sont impliquées au niveau de la synthèse des neurotransmetteurs.

9. Utilisation d'un ARNm modifié selon l'une des revendications 1 à 7, **caractérisé en ce que** l'ARNm code pour un peptide ou protéine qui se lient à des récepteurs de surface cellulaire.

10. Utilisation d'un ARNm modifié selon l'une des revendications 1 à 7 ou 9, **caractérisé en ce que** l'ARNm code pour les facteurs de croissance ou les hormones de croissance.

11. Utilisation d'un ARNm modifié selon l'une des revendications 7 ou 9, **caractérisé en ce que** l'ARNm code pour l'activateur de plasminogène tissulaire, l'insuline, les interférones, GM-CSF, l'érythropoïétine, la tyrosine hydroxylase, la DOPA décarboxylase, ou l'α-1-antitrypsine.

12. Utilisation d'un ARNm modifié selon l'une des revendications 1 à 7, **caractérisé en ce que** l'ARNm est associée à un peptide ou une protéine cationique, ou est lieé à ces derniers, en particulier le protamine, le poly-L-lysine, ou les histones.

13. Utilisation d'un ARNm modifié selon l'une des revendications 1 à 9, **caractérisé en ce que** l'ARNm code en outre pour au moins une cytokine.

14. Utilisation d'un ARNm modifié selon l'une des revendications 1 à 13, combiné à un support et/ou à un véhicule pharmacologiquement compatible.

15. Utilisation d'un ARNm modifié selon l'une des revendications 1 à 14, pour la régénération des tissues ou pour la traitement des maladies dégénératives, en particulier de maladies neurodégénératives.

16. Utilisation d'un ARNm modifié selon l'une des revendications 1 à 14, pour la traitement de la maladie d'Alzheimer, la maladie de Parkinson, ou l'arthrose.

17. Utilisation d'un ARNm modifié selon la revendication 15 ou 16, **caractérisé en ce que** l'ARNm code pour des facteurs de croissance de la famille TGF-β.

18. Utilisation d'un ARNm modifié selon la revendication 15 ou 16, **caractérisé en ce que** l'ARNm code pour EGF, FGF, PDGF, BMP, GDNF, BDNF, GDF et des facteurs neurotrophes comme NGF ou les neutrophines.

19. ARNm modifié codant pour au moins un peptide ou polypeptide biologiquement actif lequel n'est pas formé ou est formé seulement de façon insuffisante ou erronée, chez le patient à traiter, **caractérisé en ce que** la teneur en G/C de la région de l'ARNm modifié codant pour le peptide ou polypeptide est augmentée par rapport à la teneur en G/C de la région codante de l'ARNm de type sauvage codant pour le peptide ou polypeptide, et **en ce que** la séquence d'acide aminés codée est inchangée par rapport au type sauvage.

20. ARNm modifié selon la revendication 19, **caractérisé en ce que** l'ARNm modifié code pour des enzymes qui manquent ou bien sont défectueuses dans les maladies métaboliques, ou les enzymes qui sont impliquées au niveau de la synthèse des neurotransmetteurs.

21. ARNm modifié selon la revendication 19, **caractérisé en ce que** l'ARNm modifié code pour des récepteurs de surface cellulaire.

22. ARNm modifié selon la revendication 19, **caractérisé en ce que** l'ARNm code pour des protéines qui se lient à des récepteurs de surface cellulaire ou qui agissent de façon extracellulaire, choisi dans le groupe consistant en l'activateur de plasminogène tissulaire, les hormones de croissance, l'insuline, les interférones, le facteur de stimulation de colonies de granulocytes et de macrophages, les facteurs de croissance, et l'érythropoïétine,

23. Composition pharmaceutique contenant un ARNm modifié selon une des revendications 19 à 22 combiné à un support et/ou à un véhicule pharmacologiquement compatible.
